# EUROPÄISCHE PATENTSCHRIFT

(11) **EP 2 627 237 B1**
(45) Veröffentlichungstag und Bekanntmachung des Hinweises auf die Patenterteilung: **24.08.2022**
(21) Anmeldenummer: 11787630.0
(22) Anmeldetag: 17.10.2011
(51) Int. Cl.: A61B 1/00, G01C 25/00, A61B 5/06

(54) **VERFAHREN UND VORRICHTUNG ZUM KALIBRIEREN EINES OPTISCHEN SYSTEMS, ABSTANDSBESTIMMUNGSVORRICHTUNG SOWIE OPTISCHES SYSTEM**
METHOD AND DEVICE FOR CALIBRATING AN OPTICAL SYSTEM, DISTANCE DETERMINING DEVICE, AND OPTICAL SYSTEM
PROCÉDÉ ET DISPOSITIF D'ÉTALONNAGE D'UN SYSTÈME OPTIQUE, DISPOSITIF DE MESURE DE DISTANCE ET SYSTÈME OPTIQUE

(30) Priorität: 15.10.2010 DE 102010042540
(43) Veröffentlichungstag der Anmeldung: 21.08.2013
(73) Patentinhaber: Stryker European Operations Limited, Carrigtwohill, Co. Cork T45 HX08 (IE)
(72) Erfinder: KOSMECKI, Bartosz, 12047 Berlin (DE); REUTTER, Andreas, 10439 Berlin (DE); ÖZBEK, Christopher, 10823 Berlin (DE)
(74) Vertreter: Röthinger, Rainer
(86) Internationale Anmeldenummer: PCT/EP2011/068096
(87) Internationale Veröffentlichungsnummer: WO 2012/049326

(56) Entgegenhaltungen:
- EP-A1- 1 563 799
- EP-A2- 0 904 735
- EP-A2- 1 279 376
- WO-A1-01/35849
- WO-A1-01/74266
- WO-A2-2007/068017
- DE-A1-102005 012 295
- DE-A1-102005 047 481
- US-A- 4 271 829
- US-A- 4 958 932
- "Computer Assisted Head and Neck, and ENT Surgery", INTERNATIONAL JOURNAL OF COMPUTER ASSISTED RADIOLOGY AND SURGERY, Bd. 3, Nr. S1, 1. Juni 2008 (2008-06-01), Seiten 79-85, XP55018582, ISSN: 1861-6410, DOI: 10.1007/s11548-008-0177-x

## Beschreibung

Die Erfindung betrifft ein Verfahren zum Kalibrieren eines optischen Systems gemäß Anspruch 1 sowie eine Kalibriervorrichtung gemäß Anspruch 14.

Aus dem Stand der Technik sind optische Systeme mit einer Abstandsbestimmungsvorrichtung bekannt, die basierend auf dem Prinzip der Triangulation mittels kollimierter Strahlung (insbesondere Laserstrahlung) Distanzen, insbesondere Abstände des optischen Systems zu einem Objekt, ermitteln können. Wird zusätzlich zu dem Abstand auch die räumliche Lage des optischen Systems ermittelt, kann die räumliche Position des Bereiches des Objektes, auf dem die kollimierte Strahlung auftrifft, bestimmt werden.

Die DE 10 2005 012 295 A1 offenbart ein Verfahren zur endoskopischen Navigation unter Verwendung eines ein Endoskop und eine Kamera umfassenden optischen Systems, wobei zur Korrektur einer intrinsischen Verzerrung der Kamera eine Mehrzahl mit der Kamera generierter Aufnahmen miteinander verrechnet werden.

Des Weiteren beschreibt die WO 2007/068017 A2 ein medizinisches Navigationssystem mit einem Erfassungssystem zur Erfassung der räumlichen Lage eines medizinischen Instrumentes sowie mit einer Abstandsmesseinrichtung zur berührungslosen Abstandsmessung.

Aus der WO 01/35849 A1 ist zudem ein Endoskop mit einer Abstandsbestimmungsvorrichtung, die einen Triangulationslaser umfasst, bekannt.

Das von der vorliegenden Erfindung zu lösende Problem, besteht darin, ein Verfahren und eine Vorrichtung anzugeben, die ein möglichst einfaches und schnelles Kalibrieren einer Abstandsbestimmungsvorrichtung eines optischen Systems ermöglichen.

Diese Probleme werden durch das Verfahren mit den Merkmalen des Anspruchs 1 sowie durch dieVorrichtung mit den Merkmalen des Anspruchs 14 gelöst. Weiterbildungen der Erfindung sind in den abhängigen Ansprüchen angegeben.

Danach wird ein Verfahren zum Kalibrieren einer Abstandsbestimmungsvorrichtung zum Bestimmen eines Abstandes oder eines Ortsvektors zwischen einem optischen System und einem Objekt bereitgestellt, mit den Schritten:
a) Bereitstellen eines Erfassungssystems, das eine Mehrzahl von Markierungselementen sowie eine Messkamera zum Erfassen der räumlichen Position der Markierungselemente umfasst;
b) Anordnen der Markierungselemente an dem optischen System und der Abstandsbestimmungsvorrichtung;
c) Bereitstellen eines Trägers, mit dem ein Kalibriermuster verbunden ist und/oder der ein Kalibriermuster ausformt;
d) Projizieren einer Lichtstruktur auf den Träger mittels der Abstandsbestimmungsvorrichtung; wobei die Abstandsbestimmungsvorrichtung Mittel zum Projizieren aufweist, mit denen die Lichtstruktur auf den Träger projiziert wird;
e) Erfassen der Markierungselemente und (insbesondere gleichzeitiges) Erfassen des Kalibriermusters und/oder an dem Träger angeordneter Trägermarkierungselemente mittels der Messkamera;
f) Ermitteln der räumlichen Position der Markierungselemente und damit des optischen Systems unter Verwendung von Daten der Messkamera;
g) Ermitteln von Koordinaten des Kalibriermusters in einem ersten, der Messkamera zugeordneten Koordinatensystem unter Verwendung von Daten der Messkamera;
h) Erzeugen eines Bildes des Kalibriermusters und der Lichtstruktur mit einer Kamera des optischen Systems;
i) Ermitteln von Koordinaten des Bildes des Kalibriermusters und der Lichtstruktur in einem zweiten, der Bildebene der Kamera zugeordneten Koordinatensystem; und
j) Kalibrieren der Abstandsbestimmungsvorrichtung unter Verwendung der ermittelten Koordinaten sowie der ermittelten räumlichen Position des optischen Systems; wobei die Koordinaten der Lichtstruktur in dem ersten Koordinatensystem anhand der Koordinaten des Kalibriermusters in dem ersten Koordinatensystem, der Koordinaten des Kalibriermusters in dem zweiten Koordinatensystem und der Koordinaten der Lichtstruktur in dem zweiten Koordinatensystem bestimmt werden.

Die Abstandsbestimmungsvorrichtung ist insbesondere in vorgebbarer (bekannter) räumlicher Beziehung zu dem optischen System angeordnet, beispielsweise ist sie starr mit dem optischen System verbunden. Das Erfassungssystem (das z.B. in Form eines klinischen Messsystems, insbesondere eines klinischen Navigationssystems, oder als Bestandteil eines klinischen Navigationssystems ausgebildet ist) ist ein Detektionssystem, das unter Verwendung der Markierungselemente und der Messkamera eine Bestimmung der Lage eines (insbesondere medizinischen) Instrumentes ermöglicht, beispielsweise eines optischen Systems (z.B. eines Endoskops). Dabei basiert die Messkamera des Erfassungssystems insbesondere auf dem Prinzip einer Stereokamera, d.h. sie weist beispielsweise zwei zueinander beabstandete Sensorelemente (insbesondere CCD-Chips) auf, die von einem Gegenstand (insbesondere von einem Markierungselement des Erfassungssystems) Licht unter unterschiedlichen Winkeln und zu unterschiedlichen Zeitpunkten empfangen, so dass die räumliche Position des Objektes aus den Daten der Sensorelemente rekonstruiert werden kann.

Als Markierungselemente können aktive (selbst leuchtende) Elemente, wie z.B. LEDs, oder passive (nicht selbstleuchtende) Elemente, wie z.B. reflektierende Kugeln, Folien oder spezielle Muster (flache Zielmarken, Lasergravuren oder natürliche Muster wie Ecken und Kanten), verwendet werden. Es ist insbesondere bei Verwendung passiver Markierungselemente möglich, dass die Messkamera Lichtpulse, insbesondere im infraroten Wellenlängenbereich, aussendet, die von den Markierungselementen reflektiert und von der Messkamera wieder detektiert werden.

Somit können die Raumpositionen der Markierungselemente, d.h. ihre Raumkoordinaten in einem vorgegebenen, der Messkamera zugeordneten Koordinatensystem, bestimmt werden. Ist die relative Position der Markierungselemente in Bezug zu dem System, an dem sie an gebracht sind, bekannt, kann aus den Raumpositionen der Markierungselemente auf die Position des Systems geschlossen werden, wobei mit dem Begriff "Position" auch die Orientierung des Systems gemeint ist.

Das auf dem Träger angeordnete Kalibriermuster besteht zumindest teilweise aus Markierungselementen, die von der Messkamera erfasst werden können, so dass durch Erfassen des Kalibriermusters durch die Messkamera unmittelbar die räumliche Position des Kalibriermusters bzw. zumindest von Teilen des Kalibriermusters ermittelbar ist. Gemäß der Erfindung sind zusätzlich von der Messkamera erfassbare Markierungselemente (starr) an dem Träger angeordnet (Trägermarkierungselemente), so dass bei bekannter Position des Kalibriermusters relativ zu den Markierungselementen deren Raumposition (Raumkoordinaten) im Koordinatensystem der Messkamera über die Position der Markierungselemente bestimmt werden kann.

Durch das Anordnen von Markierungselementen (die z.B. ähnlich oder identisch wie die am optischen System angeordneten Markierungselemente ausgebildet sind) an dem Träger ist es möglich, ein im Prinzip beliebiges Kalibriermuster vorzusehen, das zwar von der der Abstandsbestimmungsvorrichtung zugeordneten Kamera (die insbesondere Bestandteil des optischen Systems ist, an dem die Abstandsbestimmungsvorrichtung angeordnet ist), nicht jedoch unbedingt von der Messkamera erfassbar sein muss. Beispielsweise sind an dem Träger mindestens drei Markierungselemente angeordnet, von denen mindestens drei von der der Abstandsbestimmungsvorrichtung zugeordneten Kamera erfasst werden.

Die Messkamera des Erfassungssystems wird so angeordnet und ausgerichtet, dass sie nicht nur die an dem zu kalibrierenden optischen System angebrachten Markierungselemente erfasst, sondern gleichzeitig auch das Kalibriermuster und/oder die oben erwähnten, an dem Träger angebrachten Trägermarkierungselemente. Analog zur Bestimmung der Positionen der an dem optische System angeordneten Markierungselemente können somit unter Verwendung der Messkamera gleichzeitig auch die Raumkoordinaten des Kalibriermusters ermittelt werden. Beispielsweise weist das Kalibriermuster ein Punkteraster auf, das von der Messkamera mit erfasst wird. Somit werden über die Messkamera (insbesondere zu ein und demselben Zeitpunkt) sowohl die räumliche Position des optischen Systems als auch die Koordinaten des Kalibriermusters (bzw. zumindest von Teilbereichen des Kalibriermusters) ermittelt.

Messkameras (die z.B. Bestandteil eines klinischen Messsystems, speziell eines klinischen Navigationssystems sind) zum Erfassen der Position der Markierungselemente sind an sich bekannt, so dass sie an dieser Stelle nicht weiter erläutert werden sollen. Ein Beispiel einer kommerziell erhältlichen (und im Prinzip für die Durchführung des erfindungsgemäßen Verfahrens geeigneten) Messkamera ist das System "CamBar B2" der AXIOS 3D Services GmbH. Die Erfindung ist jedoch selbstverständlich nicht auf die Verwendung einer bestimmten Art oder eines bestimmten Typs einer Messkamera beschränkt.

Mit dem optischen System, dem die zu kalibrierende Abstandsbestimmungsvorrichtung zugeordnet (und an dem sie z.B. befestigt ist), wird ein Bild des Kalibriermusters und der projizierten Lichtstruktur aufgenommen (insbesondere gleichzeitig mit dem Bestimmen der räumlichen Position des Kalibriermusters und des optischen Systems mittels der Messkamera des Erfassungssystems), z.B. weist das optische System eine CCD-Kamera auf, mit der ein Bild (insbesondere in Form eines Videobildes) der Kalibrierstruktur und der erzeugten Lichtstruktur aufgenommen wird.

Die zu kalibrierende Abstandsbestimmungsvorrichtung des optischen Systems, mit der sich ein Abstand zwischen einem dem abzubildenden Objekt zuzuwendenden Abschnitt des optischen Systems und dem Objekt bestimmen lässt, weist Mittel (insbesondere in Form eines Lasers) zum Projizieren der Lichtstruktur auf das abzubildende Objekt auf und wirkt mit einer Abbildungsoptik und einer Kamera des optischen Systems zusammen.

Die Mittel zum Projizieren sind insbesondere so in Bezug auf eine Abbildungsoptik des optischen Systems (z.B. in Form eines Endoskops) zum Abbilden eines Objektes in die Bildebene einer Kamera des optischen Systems (die die "der Abstandsbestimmungsvorrichtung zugeordnete" Kamera ist) angeordnet, dass zwischen dem durch die Mittel zum Projizieren ausgesandten Lichtbündel und dem reflektierten Lichtbündel, das von dem Objekt in die Abbildungsoptik des optischen Systems fällt, ein Winkel (z.B. 30°) besteht, so dass die Position des Bildes des auf dem Objekt erzeugten Lichtmusters in der Bildebene der Kamera von dem Abstand des Objektes zu dem optischen System abhängt. Insbesondere ist auch denkbar, dass mit Hilfe der Abstandsbestimmungsvorrichtung ein Ortsvektor zwischen einem Punkt des optischen Systems und einem Punkt der von der Abstandsbestimmungsvorrichtung projizierten Lichtstruktur bestimmt wird, d.h. es wird nicht nur der Abstand, sondern auch die Orientierung einer Verbindungslinie zwischen diesen Punkten ermittelt.

Das optische System kann auch ein Mikroskop sein, wobei die Abstandsbestimmungsvorrichtung insbesondere einen Lasertriangulationssensor aufweist, der eine Lichtstruktur erzeugt, die zur Kalibrierung im Sichtfeld des Mikrokops auf das abzubildende Objekt projiziert wird. Hierzu werden insbesondere an dem Lasertriangulationssensor Markierungselemente angeordnet, über die seine räumliche Lage ermittelbar ist. Des Weiteren wird ein Kalibrier muster auf einem Träger bereitgestellt, dessen räumliche Lage über Trägermarkierungselemente ebenfalls ermittelbar ist. Der Lasertriangulationssensor wird starr so mit dem Mikroskop verbunden, dass eine von ihm projizierte Lichtstruktur sowie das Kalibriermuster über eine Kamera des Mikroskops abgebildet werden kann. Anhand dieses Bildes sowie der ermittelten Lagen des Kalibriermusters und des Lasertriangulationssensor erfolgt dann die Kalibrierung.

Gemäß dem erfindungsgemäßen Verfahren werden einerseits Koordinaten des Kalibriermusters in einem ersten (im Prinzip beliebigen) Koordinatensystem der Messkamera und andererseits Koordinaten des Bildes des Kalibriermusters und der Lichtstruktur in einem zweiten Koordinatensystem bestimmt. Das zweite Koordinatensystem ist z.B. ein 2-D-Koordinatensystem, das sich entlang der Bildebene der Kamera des zu kalibrierenden optischen Systems erstreckt. Beispielsweise befindet sich in der Bildebene des optischen Systems ein CCD-Chip, wobei sich der Ursprung des zweiten Koordinatensystems z.B. in der Ebene des CCD-Chips befindet. Es ist auch denkbar, als zweite Koordinaten ein Paar von Pixelnummern, die die lichtempfindlichen Pixel des CCD-Chips kennzeichnen, anzugeben.

Allerdings ist auch möglich, dass das erste Koordinatensystem durch das Kalibriermuster erzeugt wird und die Koordinaten der projizierten Lichtstruktur (z.B. ein Lichtpunkt oder mehrere Lichtpunkte) in Bezug auf diese Koordinaten ermittelt werden. Analog kann das zweite Koordinatensystem durch das Bild des Kalibriermusters in der Bildebene der Kamera definiert sein, wobei die Koordinaten des Bildes der Lichtstruktur in Bezug auf diese Koordinaten angegeben werden. Beispielsweise besteht das Kalibriermuster aus einem Raster von Punkten (oder sonstigen Strukturen) mit konstantem Abstand zueinander, wobei die Punkte des Rasters das erste Koordinatensystem bilden.

Die Ermittlung der Koordinaten der projizierten Lichtstruktur erfolgt nun in Bezug auf diese Koordinaten, wobei die Ermittlung der Koordinaten insbesondere unter Ermittlung des Abstandes zu den nächstgelegenen Punkten des Rasters erfolgt. Analog erfolgt die Ermittlung der Koordinaten des Bildes der Lichtstruktur, wobei das Bild des Punkterasters das zweite Koordinatensystem festlegt. Insbesondere durch Abbildungsfehler (Verzerrungen) der Abbildungsoptik des optischen Systems kann das Punkteraster verzerrt werden, so dass das Bild des Punkterasters ein krummliniges Koordinatensystem erzeugt. Es wird darauf hingewiesen, dass das Kalibriermuster nicht unbedingt in Form eines gleichmäßigen Punkterasters realisiert werden muss. Es kann vielmehr auch ein unregelmäßiges Muster von Lichtstrukturen (z.B. Punkten) aufweisen, das das erste Koordinatensystem definiert.

Es wird ergänzend darauf hingewiesen, dass unter dem Begriff "Kalibrieren" insbesondere ein Justieren oder Kalibrieren gemäß der Definition der Norm DIN 1319 verstanden wird.

Eine "Kalibrierung" ist in der Norm DIN 1319 wie folgt definiert: "Ermitteln des Zusammenhangs zwischen Messwert oder Erwartungswert der Ausgangsgröße und dem zugehörigen wahren oder richtigen Wert der als Eingangsgröße vorliegenden Messgröße für eine betrachtete Messeinrichtung bei vorgegebenen Bedingungen. Bei der Kalibrierung erfolgt kein Eingriff, der das Messgerät verändert".

Der Begriff "Justierung" wird in der Norm DIN 1319 wie folgt definiert: "Einstellen oder Abgleichen eines Messgerätes, um systematische Messabweichungen so weit zu beseitigen, wie es für die vorgesehene Anwendung erforderlich ist. Justierung erfordert einen Eingriff, der das Messgerät bleibend verändert."

Gemäß einer weiteren Ausgestaltung der Erfindung befindet sich das optische System während der Schritte d) bis i) in einer ersten Position und wird nach Durchführen der Schritte d) bis i) aus der ersten in eine zweite Position gebracht, wobei das optische System in der zweiten Position z.B. einen anderen Abstand zu dem Träger mit dem Kalibrier- muster aufweist als in der ersten Position. Für die zweite Position werden die Schritte d) bis i) abermals durchgeführt. Für die erste und die zweite Position werden jeweils erste Koordinaten des Kalibriermusters in dem ersten Koordinatensystem und zweite Koordinaten des Bildes des Kalibriermusters und der Lichtstruktur in dem zweiten Koordinatensystem bestimmt, wobei das optische System anhand dieser Koordinaten unter Berücksichtigung der jeweils ermittelten räumlichen Position des optischen Systems kalibriert wird. Im Folgenden werden unter den "ersten Koordinaten" jeweils die Koordinaten (des Kalibriermusters und/oder der Lichtstruktur) im ersten Koordinatensystem (d.h. die jeweiligen Raumkoordinaten) und unter den "zweiten Koordinaten" die Koordinaten im zweiten Koordinatensystem (d.h. die Bildkoordinaten) verstanden.

Mit anderen Worten wird das zu kalibrierende optische System zunächst in die erste Position bewegt und es werden in dieser Stellung Koordinaten des Kalibriermusters und Koordinaten des Bildes des Kalibriermusters und der Lichtstruktur ermittelt. Anschließend wird das optische System aus der ersten Position in eine zweite Position bewegt. Mit dem in der zweiten Position befindlichen optischen System wird wiederum ein Bild des Kalibriermusters und wie für die erste Position die Koordinaten des Kalibriermusters sowie die Koordinaten des Bildes des Kalibriermusters und der Lichtstruktur ermittelt. Darüber hinaus werden jeweils auch die Koordinaten der Lichtstruktur in dem ersten, räumlichen Koordinatensystem ermittelt, wobei dies anhand der ersten und zweiten Koordinaten des Kalibriermusters und der zweiten Koordinaten der Lichtstruktur erfolgt.

Dadurch, dass die Lage des Trägers mit dem Kalibriermuster über die an dem Träger angebrachten Markierungselemente bestimmt werden kann, können, wie erwähnt, auch die räumlichen (ersten) Koordinaten von z.B. Punkten des Kalibriermusters bestimmt werden. Darüber hinaus werden die Bildkoordinaten (die zweiten Koordinaten) der Punkte (oder auch nicht punktförmiger Strukturen) des Kalibriermusters bestimmt, so dass eine Zuordnung der Bildkoordinaten der Punkte des Kalibriermusters zu den räumlichen Koordinaten der Punkte des Kalibriermusters erfolgen kann. Da auch die Bildkoordinaten der Lichtstruktur bekannt sind, ist auch ihre Lage im zweiten Koordinatensystem relativ zur Lage der Punkte des Kalibriermusters ermittelbar. Somit ist es möglich, durch Interpolation der räumlichen Koordinaten der Punkte des Kalibriermusters die räumlichen Koordinaten der Lichtstruktur zu bestimmen. Beispielsweise bilden die Strukturen (z.B. Punkte) des Kalibriermusters ein Raster, wobei z.B. der Abstand des Bildes der Lichtstruktur zu den benachbarten Bildern der Strukturen des Kalibriermusters bestimmt wird. Die den benachbarten Bildern der Kalibriermusterstrukturen entsprechenden (am Träger angeordneten) Kalibriermusterstrukturen werden identifiziert und deren räumliche (erste) Koordinaten bestimmt. Schließlich wird zwischen diesen ersten Koordinaten der Kalibriermusterstrukturen interpoliert und es werden auf Basis dieser Interpolation und der Abstände des Bildes der Lichtstruktur von den Bildern der benachbarten Kalibriermusterstrukturen die räumlichen Koordinaten der Lichtstruktur bestimmt.

Allgemein kann zur Bestimmung der räumlichen Koordinaten der Lichtstruktur eine an die ersten und zweiten Koordinaten des Kalibriermusters angepasste Näherungsabbildung ermittelt werden, mit der erste Koordinaten (Bildkoordinaten) in zweite Koordinaten (räumliche Koordinaten) abgebildet werden können. Mit dieser Näherungsabbildung werden dann zu den Bildkoordinaten der Lichtstruktur deren räumliche Koordinaten bestimmt.

Darüber hinaus ist denkbar, dass für die unterschiedlichen Positionen des optischen Systems die ersten Koordinaten der Lichtstruktur durch eine erste Näherungsfunktion (z.B. in Form eines ersten Polynoms) und/oder die zweiten Koordinaten der Lichtstruktur durch eine zweite Näherungsfunktion (z.B. in Form eines zweiten Polynoms) dargestellt werden, wobei die Näherungsfunktionen z.B. einen Abstand zwischen der projizierten Lichtstruktur und dem optischen System auf die ersten bzw. zweiten Koordinaten abbilden. Zudem kann eine Übertragungsfunktion zum Abbilden der ersten Näherungsfunktion auf die zweite Näherungsfunktion bestimmt werden, wobei mit Hilfe dieser Übertragungsfunktion aus den zweiten Koordinaten der Lichtstruktur die räumlichen Koordinaten der zugehörigen, auf den Träger projizierten Lichtstruktur ermittelbar sind. Gemäß einer anderen Weiterbildung der Erfindung wird die erste Näherungsfunktion bestimmt und das optische System in mindestens eine weitere Position gebracht, wobei das Kalibrieren gemäß Schritt j) für die mindestes eine weitere Position die folgenden Schritte umfasst:
i. Bestimmen der zweiten Koordinaten der Lichtstruktur,
ii. Bestimmen einer Geraden ("Sichtstrahlgerade") im ersten Koordinatensystem, die durch die Position der Lichtstruktur in der Bildebene des optischen Systems und durch ein optisches Zentrum des optischen Systems verläuft, und
iii. Bestimmen der ersten Koordinaten der auf den Träger projizierten Lichtstruktur durch Ermitteln der Koordinaten des Schnittpunktes der Geraden mit der ersten Näherungsfunktion.

Beispielsweise ist die erste Näherungsfunktion als Gerade ("Lichtgerade", die durch die für die unterschiedlichen Positionen des optischen Systems bestimmten ersten Koordinaten der Lichtstruktur hindurch verläuft) darstellbar und die Koordinaten der auf den Träger projizierten Lichtstruktur in dem ersten Koordinatensystem werden durch Ermitteln der Koordinaten des Schnittpunktes der gemäß Schritt ii) bestimmten Geraden mit der die erste Näherungsfunktion darstellenden Geraden oder durch Ermitteln der Koordinaten eines Punktes (z.B. eines Mittelpunktes) eines Gemeinlots in Bezug auf die beiden Geraden bestimmt. Die Sichtstrahlgerade kann insbesondere auch in Abhängigkeit weiterer optischer Eigenschaften des optischen Systems bestimmt werden, z.B. in Abhängigkeit von dessen Verzerrungseigenschaften.

Für die Kalibrierung der Abstandsbestimmungsvorrichtung werden schließlich sowohl die für die erste Position als auch die für die zweite Position des optischen Systems ermittelten Koordinaten verwendet. Natürlich kann das optische System in mehr als zwei Positionen gebracht werden, wobei für jede dieser Positionen die Koordinaten des Kalibriermusters sowie die Koordinaten des Bildes und der Lichtstruktur ermittelt und die Kalibrierung unter Verwendung sämtlicher dieser bestimmten Koordinaten vorgenommen wird.

Das optische System, an dem die Abstandsbestimmungsvorrichtung festgelegt ist, kann z.B. während des Kalibriervorganges in der Hand gehalten und so z.B. auch von einer Position in eine andere bewegt werden. Das optische System kann natürlich auch in einem Halter angeordnet und z.B. auch in dem Halter relativ zu dem Kalibriermuster (bzw. zu dem Träger, an dem das Kalibriermuster ausgebildet ist) bewegt werden.

Das optische System wird während des Kalibriervorganges z.B. in unterschiedlichen Abständen (z.B. zwischen 0,5 und 20 mm und z.B. in 0,1 mm Schritten) zu dem Träger mit dem Kalibriermuster positioniert, wobei es z.B. entlang einer Geraden bewegt (beispielsweise entlang einer Führung verschoben) wird. Zusätzlich kann das optische System auch unter unterschiedlichen Winkeln zum Träger ausgerichtet werden, wobei z.B. für jeden Winkel verschiedene Abstände des optischen Systems zum Träger durchgefahren werden. Denkbar ist auch, dass zusätzlich mehrere Träger mit unterschiedlichen Oberflächenbeschaffenheiten verwendet werden.

Beispielsweise sind die mit der Messkamera ermittelten Koordinaten, wie oben bereits erwähnt, Raumkoordinaten, d.h. sie weisen jeweils drei Koordinatenwerte auf, während die Koordinaten des Bildes des Kalibriermusters z.B. Flächenkoordinaten sind, die sich auf die Bildebene der Kamera des optischen Systems beziehen und jeweils nur zwei Koordinatenwerte umfassen. Dadurch, dass mehrere Positionen des optischen Systems in Bezug auf den Träger mit dem Kalibriermuster ausgemessen werden, entsteht eine Mehrzahl von 3D-2D Koordinatenpaaren, die zur Kalibrierung des optischen Systems herangezogen werden.

Die Kalibrierung des optischen Systems unter Verwendung der ermittelten Koordinaten kann z.B. auch darin bestehen, eine Kalibriertabelle (Look-up table) zu erstellen, in der die für jede Position des optischen Systems relativ zu dem Kalibriermuster ermittelten Koordinaten der projizierten Lichtstruktur den Koordinaten des Bildes der Lichtstruktur gegenübergestellt sind. Beispielsweise enthält eine derartige Kalibriertabelle die Raumkoordinaten (im Koordinatensystem der Messkamera) der von der Abstandsbestimmungsvorrichtung erzeugten Lichtstruktur für jede gemessene Position des optischen Systems, denen jeweils die Koordinaten (im Koordinatensystem der Bildebene der Kamera des optischen Systems) des Bildes der Lichtstruktur zugeordnet sind.

Beispielsweise wird eine Kalibriertabelle erstellt, in der sowohl die für die erste und die zweite Position ermittelten ersten Koordinaten der Lichtstruktur als auch die für die mindestens eine weitere Position des optischen Systems wie oben beschrieben ermittelten ersten Koordinaten der auf den Träger projizierten Lichtstruktur den jeweiligen zweiten Koordinaten der Lichtstruktur gegenübergestellt sind.

Mit Hilfe einer derartigen Kalibriertabelle wird also einer Position des von der Abstandsbestimmungsvorrichtung erzeugten und mit dem optischen System, dem die Abstandsbestimmungsvorrichtung zugeordnet ist, abgebildeten Lichtstruktur (z.B. in Form eines einzelnen Lichtflecks) in der Bildebene des optischen Systems eine Raumposition der Lichtstruktur zugeordnet.

Die Kalibriertabelle kann zusätzlich auch Informationen bezüglich des Winkels zwischen einer optischen Achse des optischen Systems und dem Träger mit dem Kalibriermuster enthalten. Dieser Winkel wird insbesondere unter Verwendung der mit Hilfe der an dem optischen System angeordneten Markierungselemente bestimmten räumlichen Lage des optischen Systems bestimmt. Darüber hinaus kann die Kalibriertabelle auch Information über die jeweilige räumliche Lage des optischen System enthalten, z.B. dessen Position und/oder Ausrichtung, so dass die Kalibrierung für im Prinzip beliebige Orientierungen und Positionen des optischen Systems relativ zur Abstandsbestimmungsvorrichtung verwendbar ist. Ausgenommen können Positionen und Orientierungen sein, in denen der Lichtstrahl der Abstandsbestimmungsvorrichtung mit dem optischen System stets auf den gleichen Punkt des zweiten Koordinatensystems abgebildet werden.

Es ist darüber hinaus denkbar, dass mit Hilfe der ermittelten (ersten und zweiten) Koordinaten des Kalibriermusters und der ermittelten räumlichen Position des optischen Systems ein Abstand oder ein Ortsvektor zwischen dem Träger mit dem Kalibriermuster und dem optischen System bestimmt und dieser Abstand oder Ortsvektor mit der Position des Bildes der Lichtstruktur in der Bildebene des optischen Systems in Beziehung gesetzt wird (z.B. ebenfalls mit einer Kalibriertabelle).

Denkbar ist auch, dass jeweils für die erste und die zweite Position (oder auch für weitere Positionen) des optischen Systems ein Ortsvektor zwischen einem Punkt der auf den Träger projizierten Lichtstruktur und einem Punkt des optischen Systems bestimmt wird und das Kalibrieren gemäß Schritt j) das Zuordnen dieses Ortsvektors zu den ermittelten zweiten Koordinaten der Lichtstruktur (z.B. in einer Kalibriertabelle) umfasst. Hierfür werden die Koordinaten des Punktes der auf den Träger projizierten Lichtstruktur insbesondere wie oben beschrieben bestimmt, d.h. unter Verwendung der ersten und zweiten Koordinaten der Kalibrierstruktur und der zweiten Koordinaten der Lichtstruktur.

Hierzu ist z.B. die relative Position der an dem optischen System angeordneten Markierungselemente und einer dem abzubildenden Objekt zuzuwendenden Seite des optischen Systems bekannt. Beispielsweise handelt es sich bei dem optischen System um ein Endoskop, das eine Spitze aufweist, die dem abzubildenden Objekt zuzuwenden ist, und deren Lage relativ zu den an dem Endoskop angeordneten Markierungselementen bekannt ist. Daher kann durch Ermitteln der Position der Markierungselemente auf die Position der Endoskopspitze geschlossen und anhand der ermittelten Raumkoordinaten der Lichtstruktur ein Abstand zwischen der Endoskopspitze und der projizierten Lichtstruktur bestimmt werden. Auch ist möglich, dass der Betrag und/oder die Richtung eines Differenzvektors zwischen der Position der Endoskopspitze (oder eines sonstigen Referenzpunktes des optischen Systems) und der projizierten Lichtstruktur in Bezug zu den Koordinaten des Bildes der Lichtstruktur in Beziehung gesetzt werden.

Die Erfindung betrifft auch eine Kalibriervorrichtung zum Kalibrieren einer Abstandsbestimmungsvorrichtung zum Bestimmen eines Ortsvektors zwischen einem bekannten Punkt eines optischen Systems und einem markierten Punkt eines Objekts, zum Durchführen eines wie oben beschriebenen Verfahrens, mit
a) einem Träger, mit dem ein Kalibriermuster verbunden ist und/oder der ein Kalibriermuster ausformt;
b) einem Erfassungssystem, das eine Mehrzahl von an dem optischen System und/oder der Abstandsbestimmungsvorrichtung angeordneten Markierungselementen sowie eine Messkamera zum Erfassen der räumlichen Position der Markierungselemente, des Kalibriermusters und/oder an dem Träger angeordneter Trägermarkierungselemente umfasst;
c) Positionsermittlungsmitteln zum Ermitteln der räumlichen Position des optischen Systems anhand der Daten der Messkamera;
d) Koordinatenermittlungsmitteln zum Ermitteln von Koordinaten des Kalibriermusters in einem ersten, der Messkamera zugeordneten Koordinatensystem, von Koordinaten eines Bildes des Kalibriermusters und einer unter Verwendung von der Abstandsbestimmungsvorrichtung zugehörigen Mitteln zum Projizieren projizierten Lichtstruktur in einem zweiten, der Bildebene einer Kamera des optischen Systems zugeordneten Koordinatensystem; sowie
e) Kalibrierungsmitteln zum Kalibrieren der Abstandsbestimmungsvorrichtung anhand der ermittelten Koordinaten sowie der ermittelten räumlichen Position des optischen Systems, wobei die Kalibrierungsmittel ausgebildet sind, die Koordinaten der Lichtstruktur in dem ersten Koordinatensystem anhand der Koordinaten des Kalibriermusters in dem ersten Koordinatensystem, der Koordinaten des Kalibriermusters in dem zweiten Koordinatensystem und der Koordinaten der Lichtstruktur in dem zweiten Koordinatensystem zu bestimmen.

Insbesondere sind die Positionsermittlungsmittel Bestandteil des (insbesondere klinischen) Erfassungssystems und z.B. in Form einer Software oder eines entsprechend programmierten elektronischen Bauelementes realisiert. Das "Erfassungssystem" ist, wie weiter oben bereits erläutert, insbesondere in Form eines klinischen Messsystems, insbesondere in Form eines klinischen Navigationssystems, oder in Form eines Bestandteils eines klinischen Messsystems ausgebildet. Die Koordinatenermittlungsmittel und/oder Kalibrierungsmittel sind insbesondere ebenfalls in Form einer Software oder eines entsprechend programmierten elektronischen Bauelementes ausgebildet.

Auch umfasst die Kalibriervorrichtung zu dem zu kalibrierenden optischen System beabstandet anzuordnende Mittel (insbesondere einen Laser) zum Erzeugen eines Lichtmusters, wie bereits oben beschrieben.

Des Weiteren kann das Kalibriermuster auf den Träger aufgeklebt, aufgedruckt und/oder in den Träger eingeprägt sein. Auch ist möglich, dass der Träger Verbindungsmittel zum Verbinden mit dem optischen System aufweist. Insbesondere sind die Verbindungsmittel so ausgebildet, dass sie eine relative Bewegung zwischen dem Träger und dem optischen System ermöglichen, so dass unterschiedliche Abstände zwischen dem Träger und dem optischen System eingestellt werden können. Beispielsweise umfassen die Verbindungsmittel eine Führung (z.B. eine Schiene), über die das optische System geradlinig gegenüber dem Träger verschoben werden kann.

In einem Beispiel weist die Abstandsbestimmungsvorrichtung auf:
- einen ersten, in Richtung einer optischen Achse des optischen Systems verlaufenden Kanal, in dem ein Abschnitt einer Lichtleitfaser geführt ist, die zum Leiten und Aussenden von Licht in Richtung des Objektes dient; und
- einen zweiten Kanal, in dem ein weiterer Abschnitt der Lichtleitfaser geführt ist und der so verläuft, dass der weitere Abschnitt der Lichtleitfaser in Richtung auf den ersten Kanal umgebogen wird.

Die Lichtleitfaser (insbesondere in Form einer Glasfaser) ist z.B. mit einem Laser der Abstandsbestimmungsvorrichtung gekoppelt und leitet Licht des Lasers zu einer Abstrahlseite des optischen Systems. Der Kanal, in dem die Lichtleitfaser geführt ist, ist insbesondere ein länglicher Hohlkörper, z.B. in Form eines (beispielsweise metallischen) Rohres.

Die Abstandsbestimmungsvorrichtung weist z.B. einen Grundkörper auf, in dem eine Aufnahme für einen entlang der optischen Achse des optischen Systems verlaufenden Lichtführungsschaft des optischen Systems ausgebildet ist. Des Weiteren kann in dem Grundkörper eine Aussparung vorgesehen sein, die den zweiten Kanal zur Führung der Lichtleitfaser ausbildet.

Beispielsweise weist der zweite Kanal einen ersten, zweiten und dritten Abschnitt auf, wobei der zweite Abschnitt mit seinem einen Ende über eine erste Krümmung mit dem ersten Abschnitt und mit seinem anderen Ende über eine zweite Krümmung mit dem dritten Abschnitt verbunden ist. Der zweite Kanal ermöglicht somit eine Umlenkung der Lichtleitfaser bei möglichst geringen Abstrahlverlusten. Beispielsweise beträgt der Krümmungsradius der ersten und/oder der zweiten Krümmung mindestens 5 mm.

Beispielsweise umfasst das optische System:
- eine Mehrzahl von Markierungselementen zum Ermitteln der räumlichen Position des optischen Systems; und
- Verbindungsmittel, über die die Markierungselemente schwenkbar mit dem optischen System verbunden sind.

Beispielsweise sind die Markierungselemente, die z.B. wie oben beschrieben ausgebildet sind, an einem gemeinsamen Halter angeordnet, der über die Verbindungsmittel schwenkbar mit dem optischen System verbunden ist.

Gemäß einer Ausgestaltung des optischen Systems sind die Markierungselemente über die Verbindungsmittel um eine Achse schwenkbar, die parallel zu einer optischen Achse des optischen Systems verläuft. Beispielsweise handelt es sich bei dem optischen System um ein (z.B. starres) Endoskop, wobei die optische Achse des Endoskops insbesondere durch die optische Achse des optischen Abbildungssystems des Endoskops festgelegt ist. Darüber hinaus kann an dem optischen System des optischen Systems eine wie oben beschriebene Abstandsbestimmungsvorrichtung angeordnet sein.

Die Erfindung wird im Folgenden anhand von Ausführungsbeispielen unter Bezugnahme auf die Figuren näher erläutert. Es zeigen:
- Figur 1: eine Kalibriervorrichtung gemäß einem ersten Ausführungsbeispiel der Erfindung;
- Figur 2: eine Kalibriervorrichtung gemäß einem zweiten Ausführungsbeispiel der Erfindung;
- Figur 3: ein Flussdiagramm zur Erläuterung des erfindungsgemäßen Verfahrens;
- Figur 4: eine Abstandsbestimmungsvorrichtung ausgestaltet zur Anbringung an einem Endoskop;
- Figuren 5 und 6: Ausschnitte aus Figur 4 in vergrößerter Darstellung;
- Figur 7: eine perspektivische Ansicht der Abstandsbestimmungsvorrichtung aus FIG 4;
- Figur 8: eine Detailansicht des Endoskops aus FIG 7;
- Figuren 9A, 9B: verschiedene Ansichten eines Teilstücks der Abstandsbestimmungsvorrichtung des Endoskops aus den Figuren 7 und 8;
- Figur 10: eine Vorderansicht der Spitze des Endoskops aus den Figuren 7 und 8;
- Figuren 11A, 11B: eine weitere perspektivische Ansicht des Endoskops der Figuren 7 und 8 in montiertem Zustand bzw. in Explosionsdarstellung; und
- Figuren 12A bis 12C: das Endoskop der Figuren 7 und 8 für unterschiedliche Schwenkstellungen der Markierungselemente.

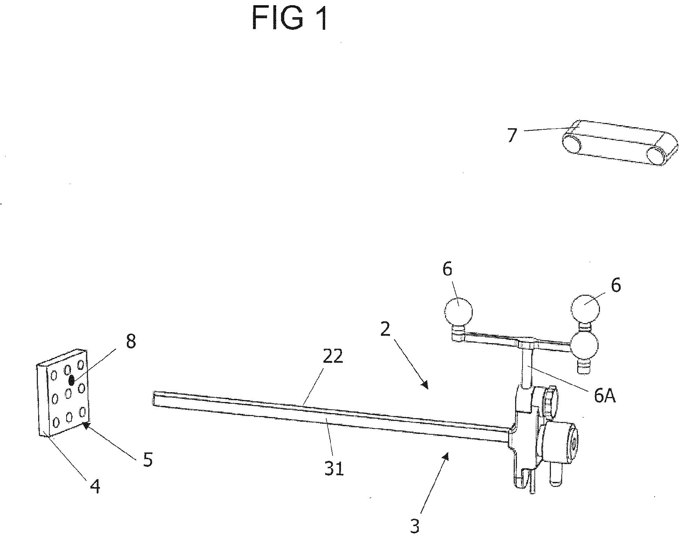

Die in Figur 1 dargestellte Kalibriervorrichtung dient zum Kalibrieren einer Abstandsbestimmungsvorrichtung 2 eines optischen Systems in Form eines Endoskops 3. Die Abstandsvorrichtung 2 weist einen Laser (nicht dargestellt) auf, dessen Licht über eine sich mit einem Teilabschnitt entlang eines Endoskopschaftes 31 erstreckende Lichtleitfaser 22 zur Spitze des Endoskops geleitet und auf das mittels des Endoskops zu untersuchende Objekt projiziert wird. Der von der Abstandsvorrichtung auf dem Objekt erzeugte Lichtfleck wird über die Abbildungsoptik und eine Kamera (nicht dargestellt) des Endoskops angebildet, wobei aus der Position des Lichtflecks in der Bildebene der Kamera der Abstand zwischen der Endoskopspitze und dem Objekt ermittelt werden kann.

Die Kalibriervorrichtung 1 umfasst einen Träger 4, mit dem ein Kalibriermuster 5 verbunden ist, und der so positioniert ist, dass mit der Abstandsbestimmungsvorrichtung ein Lichtfleck 8 auf den Träger 4 projiziert und mit der Kamera des Endoskops 3 ein Bild des Kalibriermusters 5 und des Lichtflecks 8 erzeugt werden kann. Das Kalibriermuster 5 besteht aus einer Mehrzahl von rasterartig an dem Träger angeordneten Kalibrierpunkten, die z.B. auf den Träger 4 aufgedruckt oder aufgeklebt sind.

Des Weiteren umfasst die Kalibriervorrichtung 1 ein Erfassungssystem, das mehrere Markierungselemente in Form von reflektierenden Markierungskugeln 6 umfasst. Die Markierungskugeln 6 sind über einen gemeinsamen Halter 6A mit dem Endoskop 3 verbunden. Das Erfassungssystem weist des Weiteren eine Messkamera in Form einer Stereokamera 7 auf, die so angeordnet ist, dass sie gleichzeitig die Markierungskugeln 6 und das Kalibriermuster 5 erfasst. Die Kalibriervorrichtung 1 weist zudem Positionsermittlungsmittel (nicht dargestellt), die aus den Daten der Messkamera 7 die räumliche Position (d.h. die Koordinaten eines oder mehrerer Referenzpunkte des Endoskops sowie dessen Orientierung im Raum) ermitteln. Insbesondere sind die Positionsermittlungsmittel in Form eines entsprechend programmierten Computers ausgebildet.

Die Kalibriervorrichtung weist darüber hinaus Koordinatenermittlungsmittel, die z.B. ebenfalls in Form eines Computerprogramms ausgebildet sind, auf, die anhand der Daten der Messkamera 7 erste Koordinaten in Form von Raumkoordinaten des Kalibriermusters 5 ermitteln.

Darüber hinaus bestimmen die Koordinatenermittlungsmittel auch Koordinaten des mit der Kamera des Endoskops erzeugten Bildes des Kalibriermusters 5 und des Lichtflecks 8 in einem zweiten, der Bildebene der Kamera des optischen Systems zugeordneten Koordinatensystem. Kalibrierungsmittel (die insbesondere ebenfalls als Computerprogramm ausgebildet sind) der Kalibriervorrichtung kalibrieren schließlich die Abstandsbestimmungsvorrichtung 2 des Endoskops anhand der ermittelten (ersten und zweiten) Koordinaten sowie der ermittelten räumlichen Position des optischen Systems.

Das Kalibrieren der Abstandsbestimmungsvorrichtung 2 mit Hilfe der Kalibriervorrichtung Fig.1 erfolgt derart, dass das Endoskop 3 in unterschiedliche Positionen (insbesondere Abstände) relativ zu dem Träger 4 (d.h. zu dem Kalibriermuster 5) gebracht wird und jeweils die Koordinaten des Kalibriermusters 5 im Raum bestimmt werden. Zusätzlich wird mit der Kamera des Endoskops 3 ein Bild des Kalibriermusters 5 und des Lichtflecks 8 erzeugt und mittels der Koordinatenermittlungsmittel die Koordinaten des Kalibriermusters und des Lichtflecks in der Bildebene der Kamera bestimmt. Zudem werden anhand der bekannten Raumkoordinaten des Kalibriermusters sowie der Koordinaten des Bildes des Kalibriermusters und des Bildes des Lichtflecks die Raumkoordinaten des Lichtflecks 8 bestimmt.

Gemäß dem Ausführungsbeispiel der Figur 1 besteht das Kalibriermuster 5, wie erwähnt, aus einem Raster von Kalibrierpunkten, wobei jeweils die Koordinaten der einzelnen Kalibrierpunkte bestimmt werden. Die Kalibrierungsmittel erzeugen dann eine Kalibriertabelle, in der beispielsweise für jeden Abstand des Endoskops 3 von dem Kalibriermuster 5 die räumlichen Koordinaten des Lichtflecks 8 auf dem Träger den Koordinaten des Bildes des Lichtflecks 8 gegenübergestellt sind. Somit kann nach der Kalibrierung der Abstandsbestimmungsvorrichtung (d.h. nach Fertigstellung der Kalibriertabelle) einer Position des Lichtflecks in der Bildebene der Kamera eine Raumposition des auf ein Objekt projizierten Lichtflecks zugeordnet werden. Dabei wird es vorkommen, dass die Position des Bildes des Lichtflecks zwischen in der Kalibriertabelle enthaltenen Lichtfleckpositionen liegt, so dass die zugehörige räumliche Position des Lichtflecks auf dem Objekt durch Interpolation der in der Kalibriertabelle erfassten Werte erfolgen muss.

Es ist auch denkbar, dass während des Kalibriervorganges anstelle der Raumposition des auf den Träger projizierten Lichtflecks 8 ein Abstand eines Referenzpunktes (z.B. die Spitze des Schaftes 31 des Endoskops 3) und dem Träger einer Position des Bildes des Lichtflecks zugeordnet wird. Aus der Lage des Lichtflecks in der Bildebene kann dann nach Kalibrierung der Abstandsbestimmungsvorrichtung der Abstand des Referenzpunktes des Endoskops zu dem Objekt bestimmt werden, wobei der Abstand wiederum gegebenenfalls durch Interpolation in der Kalibriertabelle erfasster Werte, die benachbart zu der tatsächlichen Position des Lichtflecks in der Bildebene liegen, ermittelt werden muss.

Es ist auch möglich, dass in der Kalibriertabelle zwar die Raumpositionen des auf dem Träger projizierten Lichtflecks 8 erfasst werden, diese jedoch nicht absolut, sondern relativ zu einem Referenzpunkt des Endoskops (wie oben bereits erwähnt z.B. in Form der Spitze des Endoskops) angegeben werden. Nach Kalibrierung der Abstandsbestimmungsvorrichtung kann dann aus der Position des Bildes des durch die Abstandsbestimmungsvorrichtung auf dem zu untersuchenden Objekt erzeugten Lichtflecks in der Bildebene der Kamera und nach Ermitteln der räumlichen Lage des Endoskops (oder eines anderen optischen Systems) die tatsächliche Lage des Lichtflecks im Raum relativ zu dem Referenzpunkt ermittelt werden.

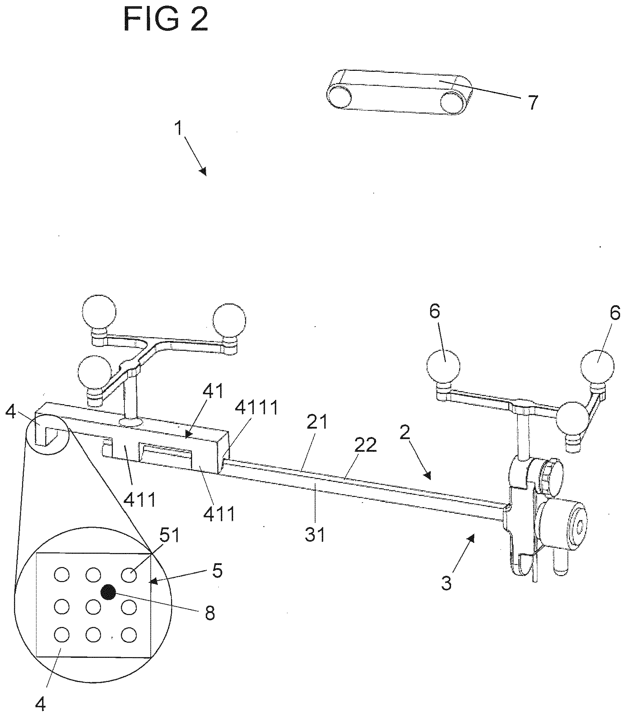

Figur 2 stellt ein weiteres Ausführungsbeispiel der erfindungsgemäßen Kalibriervorrichtung dar, die wie die Kalibriervorrichtung der Figur 1 eine Stereokamera 7 sowie ein aus mehreren rasterförmig angeordneten Kalibrierpunkten 51 bestehendes Kalibriermuster 5 umfasst. Das Kalibriermuster ist ebenfalls wie in Figur 1 mit einem Träger 4 verbunden.

Im Unterschied zur Figur 1 ist der Träger 4 mit dem Kalibriermuster 5 jedoch nicht separat zu der Abstandsbestimmungsvorrichtung 2 des Endoskops 3 angeordnet, sondern über Verbindungsmittel in Form einer Führung 41, die mit dem Schaft 31 des Endoskops 3 bewegbar gekoppelt ist. Insbesondere ist der Träger 4 einstückig mit der Führung 41 ausgebildet, wobei die Führung 41 zwei Backen 411 aufweist, in denen jeweils eine Führungsnut 4111 ausgebildet ist, die an die äußeren Abmessungen des Endoskopschaftes 31 sowie eines entlang des Endoskopschaftes 31 verlaufenden Rohres 21, in dem sich die Lichtleitfaser 22 der Abstandsbestimmungsvorrichtung 2 erstreckt, angepasst ist.

Die Führung 41 wird somit auf den Endoskopschaft 31 aufgesetzt und kann während des Kalibriervorganges entlang der optischen Achse des Endoskops, die durch den Verlauf des Schaftes 31 (in dem die Abbildungsoptik des Endoskops angeordnet ist) bestimmt ist, verschoben werden, so dass unterschiedliche Abstände zwischen dem Träger 4 mit dem Kalibriermuster 5 und der Spitzes des Endoskopschaftes 31 eingestellt werden können. Entsprechend werden während des Kalibrierens der Abstandsbestimmungsvorrichtung 2 durch Verschieben der Führung 41 auf dem Endoskopschaft 31 verschiedene Abstände eingestellt und wie oben erläutert jeweils die Koordinaten der Kalibrierpunkte 51 und die Koordinaten der jeweiligen Bilder der Kalibrierpunkte und des von der Abstandsbestimmungsvorrichtung 2 projizierten Lichtflecks 8 in der Bildebene der Kamera ermittelt und eine Kalibriertabelle erzeugt.

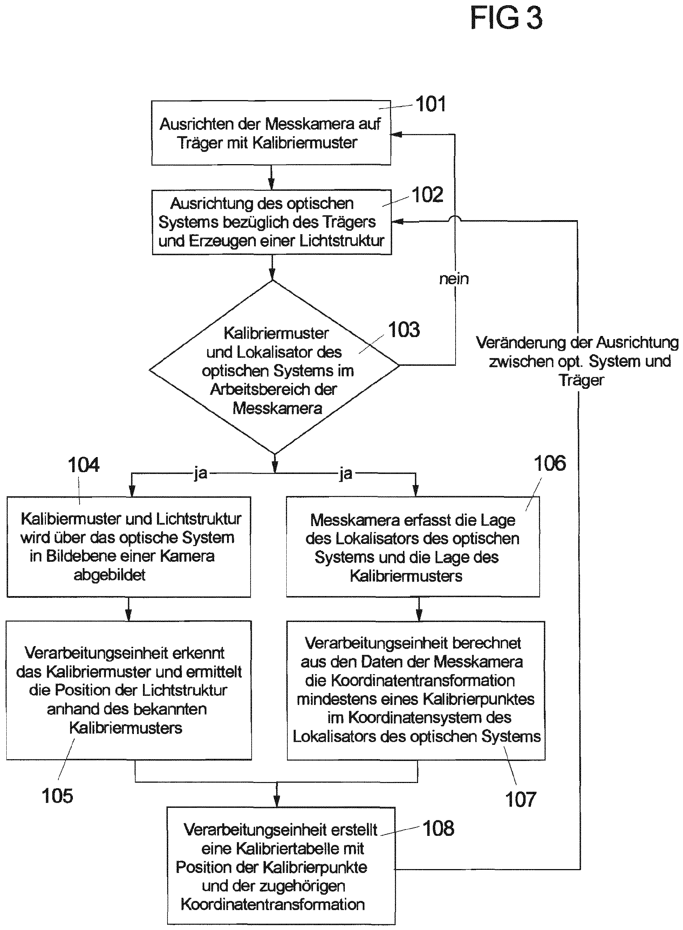

Figur 3 ist ein Flussdiagramm zur Illustration des erfindungsgemäßen Kalibrierverfahrens, das insbesondere unter Verwendung einer Kalibriervorrichtung der Figuren 1 oder 2 durchgeführt wird.

Nach Ausrichten der Messkamera auf den Träger mit dem Kalibriermuster wird das optische System bezüglich des Trägers positioniert, so dass mittels der Abstandsbestimmungsvorrichtung des optischen Systems eine Lichtstruktur auf dem Träger erzeugt und der Träger, sowie an dem optischen System angeordnete Markierungselemente von der Messkamera erfasst werden können. (Schritte 101, 102). Beispielsweise wird das optische System so ausgerichtet, dass seine optische Achse (z.B. die Achse des Endoskopschaftes, falls es sich bei dem optischen System um ein Endoskop handelt) zumindest annähernd senkrecht zum Träger orientiert ist.

Anschließend wird überprüft, ob sich das Kalibriermuster und die an dem optischen System angebrachten Markierungselemente des optischen Systems im Sichtbereich der Messkamera des Erfassungssystems befinden (Schritt 103). Ist dies der Fall, werden gemäß den Schritten 104, 105 zum einen das Kalibriermuster und die Lichtstruktur mit einer Kamera des optischen Systems (z.B. auf einen CCD-Chip der Kamera) und die (zweiten) Koordinaten des abgebildeten Kalibriermusters und der Lichtstruktur ermittelt.

Zum anderen werden mit der Messkamera die Position der Markierungselemente ("Lokalisator") und die Position des Kalibriermusters im ersten Koordinatensystem erfasst (Schritt 106), wobei die Messkamera die Lage des Kalibriermusters entweder direkt oder über einen weiteren Lokalisator ("Trägermarkierungselemente") am Träger des Kalibriermusters erfasst.

Nach Bestimmung der (ersten und zweiten) Koordinaten wird ein Eintrag in einer Kalibriertabelle erstellt, der die ermittelten Koordinaten gegenüberstellt (Schritte 107, 108). Anschließend wird das optische System in eine andere Position bewegt und es werden die Schritte 104 bis 108 wiederholt.

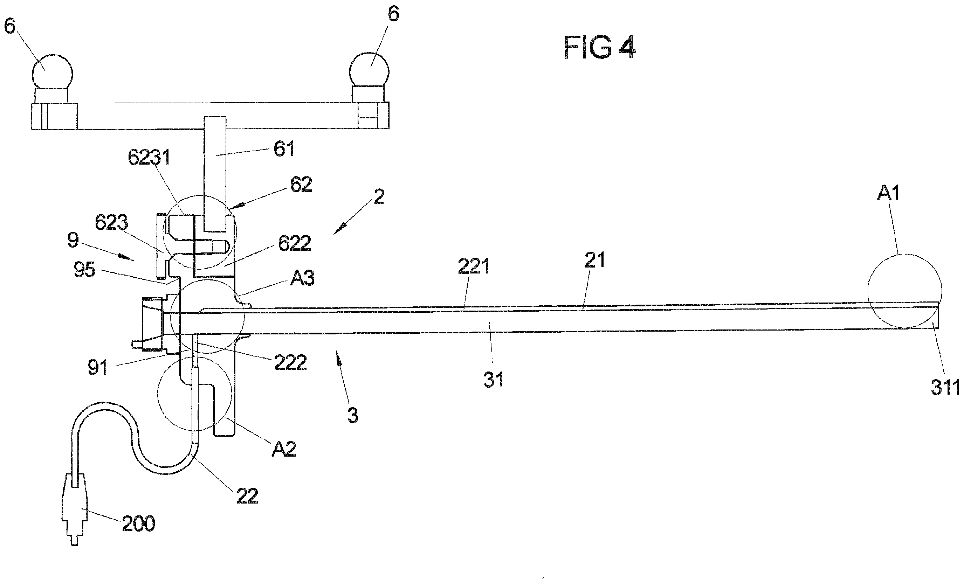

Figur 4 zeigt ein Beispiel einer Abstandsbestimmungsvorrichtung 2, die an einem Endoskop 3 angeordnet ist. Beispielsweise entsprechen das Endoskop und die Abstandsbestimmungsvorrichtung der Figuren 1 und 2 der Anordnung der Figur 4.

Die Abstandsbestimmungsvorrichtung 2 weist einen in Form eines sich entlang einer optischen Achse des Endoskops 3 erstreckenden Rohres 21 ausgebildeten ersten Kanal auf, in dem sich ein erster Abschnitt 221 einer Lichtleitfaser 22 erstreckt. Die optische Achse des Endoskops 3 wird insbesondere durch den Verlauf eines Schaftes 31 (Lichtführungsschaft) des Endoskops bestimmt, in dem sich dessen Abbildungssystem befindet. Das Rohr 21 mit der Lichtleitfaser 22 erstreckt sich so entlang des Schaftes 31, dass es an einer Außenseite des (insbesondere starren) Schaftes 31 anliegt.

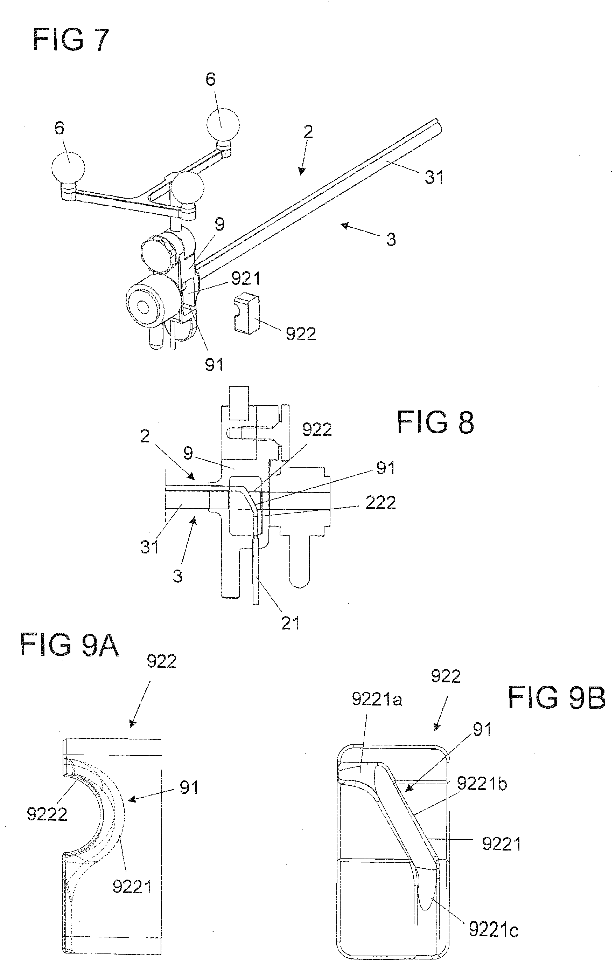

Die Abstandsbestimmungsvorrichtung 2 weist zudem einen (z.B. aus einem Metall oder einem Kunststoff gebildeten) Grundkörper 9 auf, der einen zweiten Kanal 91 ausformt, in dem ein zweiter Abschnitt 222 der Lichtleitfaser 22 verläuft und in Richtung auf das Rohr 21 umgebogen wird. Der Grundkörper 9 ist in der Figur 8 vergrößert dargestellt.

Die Lichtleitfaser 22 ist in dem Rohr 21 bis zu einer Spitze 311 des Endoskopschaftes 31 geführt. Das andere Ende der Lichtleitfaser 22, das aus dem Grundkörper 9 austritt, ist insbesondere über einen Stecker 200 mit einer Lichtquelle koppelbar.

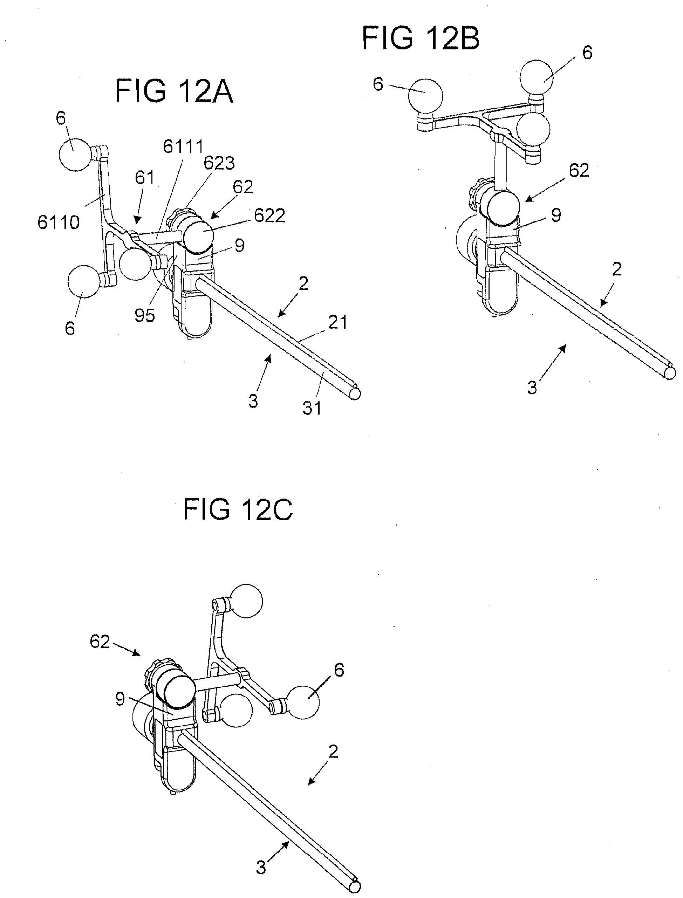

Wie bereits in den Figuren 1 und 2 gezeigt, sind an dem Endoskop 3 Markierungskugeln 6 zum Bestimmen der räumlichen Position des Endoskops 3 angeordnet. Die Markierungskugeln 6 sind über einen Halter 61, der über Verbindungsmittel 62 (Kopplungsmechanismus) schwenkbar mit dem Grundkörper 9 der Abstandsbestimmungsvorrichtung 2 gekoppelt ist, verbunden. Auf dem Kopplungsmechanismus zum schwenkbaren Verbinden der Markierungskugeln mit dem Grundkörper 9 wird weiter unten im Zusammenhang mit den Figuren 12A bis 12C genauer eingegangen.

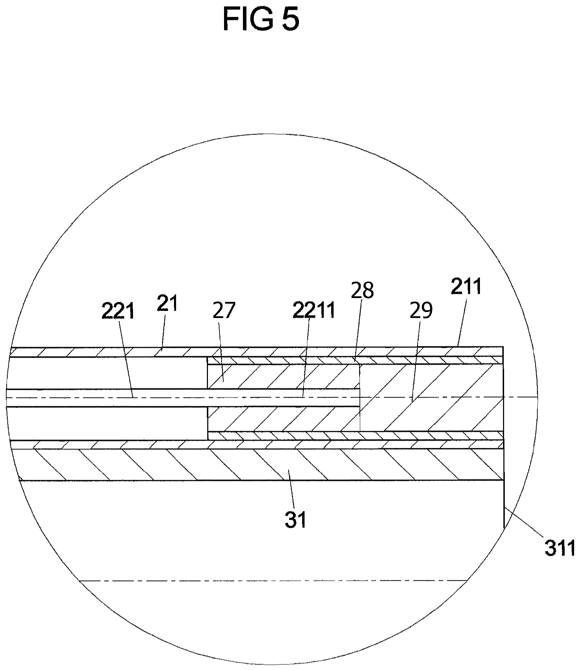

Die Figur 5 zeigt einen Ausschnitt (in Figur 5 angedeutet durch den Kreis A1) eines vorderen Bereiches (der Spitze) der Abstandsbestimmungsvorrichtung 2 mit dem Abschnitt 221 der Lichtleitfaser 22. Es ist zu erkennen, dass das Rohr 21 mit einem Abschnitt seiner Außenseite an der Außenseite des ebenfalls rohrförmig ausgebildeten Endoskopschaftes 31 anliegt. Das Ende 2211 der Lichtleitfaser 22 ist in einer Kapillare 27, die in das Rohr 21 eingesetzt ist, befestigt (z.B. eingeklebt). Die Kapillare 27 wiederum sitzt in einer Tülle 28, die einen Außendurchmesser aufweist, der in etwa dem Innendurchmesser des Rohres 21 entspricht, wobei die Kapillare 27 in die Tülle 28 eingeklebt und die Tülle ihrerseits an dem Rohr 21 festgelegt (z.B. ebenfalls eingeklebt) sein kann. Unter dem Begriff "Kapillare" und "Tülle" sind hier hohlzylindrische Strukturen gemeint, wobei die eine (die "Kapillare") einen kleineren Durchmesser als die andere (die "Tülle") besitzt. Das Ende 2211 der Lichtleitfaser 21 ist mit einer Stablinse 29 gekoppelt, die sich zwischen einem Ende der Kapillare 27 und einer Stirnseite eines Endes 211 des Rohres 21 befindet. Die Stablinse 29 ist insbesondere in Form einer Gradientenindexlinse ausgebildet.

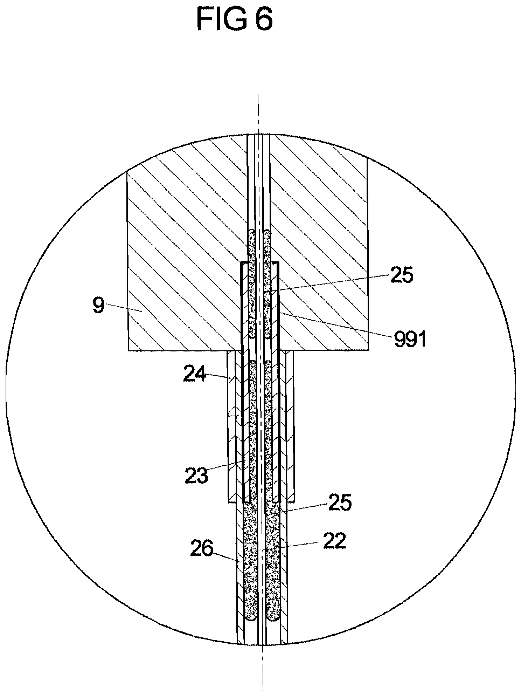

Figur 6 zeigt einen weiteren Ausschnitt (Ausschnitt A2) aus Figur 4, nämlich die Verbindung der Lichtleitfaser 22 mit dem Grundkörper 9 der Abstandsbestimmungsvorrichtung 2. Danach ist ein Teilbereich der Lichtleitfaser in ein Adapterrohr 23 eingeklebt (mittels eines Klebstoffes 25) und insbesondere zusätzlich vercrimpt (über eine Crimphülse 24). Das Adapterrohr 23 ragt in eine Schutzumhüllung (Schutzschlauch 26), die die Lichtleitfaser 22 umgibt, hinein.

Der Klebstoff 25 füllt insbesondere einen Raum zwischen der Lichtleitfaser 22 und dem Schutzschlauch 26 aus, um eine möglichst druckdichte Verbindung zwischen dem Adapterrohr und der Lichtleitfaser zu realisieren. Des Weiteren ragt ein Ende des Adapterrohres 23 in eine Bohrung 991 in dem Grundkörper 9 hinein und ist in dieser Bohrung festgelegt (insbesondere ebenfalls verklebt). Um eine möglichst druckdichte Verbindung auch zwischen der Lichtleitfaser und dem Grundkörper 9 zu erreichen, wird der Klebstoff auf den Klebflächen möglichst gleichmäßig aufgebracht.

Figur 7 zeigt eine perspektivische Darstellung der Anordnung aus Figur 4. Hierbei ist zu erkennen, dass der Grundkörper 9 im Bereich des zweiten Kanals, durch den hindurch sich der zweite Abschnitt 222 der Lichtleitfaser 22 erstreckt, eine Aussparung 921 aufweist, in die ein Führungselement 922 einsetzbar ist. Die seitliche Ansicht der Fig. 8 eines Ausschnitts (im Bereich A3 der Fig. 4) zeigt das in die Aussparung 921 eingesetzte Führungselement 922.

Sowohl in dem Grundkörper 9 (innen an die Aussparung 921 angrenzend) als auch in dem Führungselement 922 sind jeweils Ausnehmungen ausgebildet, die zusammen - wenn das Führungselement 922 in den Grundkörper eingesetzt ist - den zweiten Kanal für die Lichtleitfaser sowie eine Aufnahme für den Endoskopschaft bilden. Eine in dem Grundkörper 9 angrenzend an die Aussparung 921 ausgeformte Ausnehmung (Vertiefung, nicht dargestellt) verläuft parallel zum Schaft 31 und weist einen halbkreisförmigen Querschnitt auf. Entsprechend weist auch das Führungselement 922 eine Ausnehmung 9222 mit einem halbkreisförmigen Querschnitt auf, die entlang des Schaftes 31 orientiert ist, wenn das Führungselement 922 in den Grundkörper 9 eingesetzt ist. Somit bildet die Ausnehmung in dem Grundkörper 9 und die Ausnehmung 9222 in dem Führungselement 922 nach Einsetzen des Führungselementes eine Durchgangsöffnung durch den Grundkörper 9, durch die hindurch sich der Endoskopschaft 31 zu einem weiteren Bestandteil des Endoskops (z.B. einer Kamera oder einer sonstigen optischen oder mechanischen Komponente) erstreckt.

Des Weiteren ist in dem Führungselement 922 eine Ausnehmung 9221 gebildet, die zusammen mit einer entsprechenden Ausnehmung in dem Grundkörper 9 (innen angrenzend an die Aussparung 921, d.h. in einem Bodenbereich der Aussparung 921) den zweiten Kanal 91 zur Führung des zweiten Abschnitts 222 der Lichtleitfaser 22 ausformt. Die Ausnehmung 9221 ist ähnlich wie die halbkreisförmige Öffnung 9222 für den Endoskopschaft 31 nicht in Form einer Durchgangsöffnung, sondern zu einer Seite des Führungselementes hin offen ausgebildet, so dass der zweite Kanal erst durch Einsetzen des Führungselementes 922 in den Grundkörper komplettiert wird. Insbesondere wird der Einsatz 922 nach dem Einsetzen in die Aussparung 921 mit dem Grundkörper 9 verbunden (insbesondere verschweißt).

Der nach dem Einsetzen des Einsatzes 922 entstandene zweite Kanal 91 weist insbesondere einen ersten, zweiten und dritten Abschnitt auf, so dass die Ausnehmung 9221 in dem Führungselement 922 entsprechende Abschnitte 9221a bis 9221c aufweist. Der zweite Abschnitt 9221b (der mittlere Abschnitt) ist jeweils über eine Krümmung mit dem ersten bzw. mit dem zweiten Abschnitt 9221a, 9221c verbunden, so dass eine durch den zweiten Kanal hindurch geführte Lichtleitfaser zwar um ca. 90º umgebogen werden kann, der Biegeradius jedoch möglichst groß bleibt; beispielsweise weisen die erste und die zweite Krümmung einen Krümmungsradius von mindestens 5 mm auf.

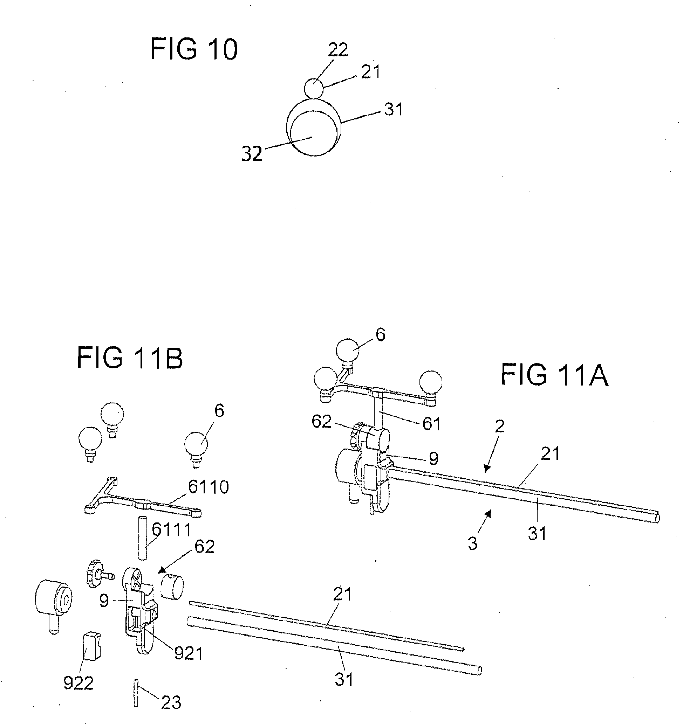

Figur 10 zeigt einen Querschnitt durch den Endoskopschaft 31 des Endoskops 3 und dem entlang des Endoskopschaftes 31 angeordneten Rohres 21, in dem der Abschnitt 221 der Lichtleitfaser 22 der Abstandsbestimmungsvorrichtung geführt ist. In dem rohrförmig ausgebildeten Endoskopschaft 31 ist eine Abbildungsoptik 32 (insbesondere in Form einer Stablinse) exzentrisch in Bezug auf den Mittelpunkt des Schaftes 31 angeordnet. Im nicht von der Abbildungsoptik 32 ausgefüllten Innenraum des Schaftes 31 können sich weitere Lichtleitfasern erstrecken, über die Licht zur Beleuchtung eines mittels des Endoskops abzubildenden Objektes geführt werden kann.

Das Rohr 21 mit der Lichtleitfaser 22 ist an einem Abschnitt der Außenseite des Endoskopschaftes 31 angeordnet, der einen möglichst großen Abstand von der Achse der Abbildungsoptik 32 (d. h. von der optischen Achse des Endoskops) aufweist, damit ein möglichst großer Abstand zwischen der Achse der Lichtleitfaser und optischen Achse des Endoskops entsteht, da die Genauigkeit der Abstandsmessung mit größer werdendem Abstand zwischen diesen beiden Achsen zunimmt.

Die Figuren 11A, 11B zeigen das Endoskop der Figuren 4 und 7 in perspektivischer (Fig. 11A) bzw. Explosionsdarstellung (Figur 11B).

Die Figuren 12A bis 12C zeigen wiederum das Endoskop der Figuren 4 und 7, jedoch für verschiedene Positionen der Markierungskugeln 6. Wie bereits zuvor erwähnt, sind die Markierungskugeln 6 über einen Halter 61, der sich aus einem T-förmigen ersten Teil 6110 und einem sich zu diesem ersten Teil senkrecht erstreckenden, stabförmigen zweiten Teil 6111 zusammensetzt, mit dem Grundkörper 9 der Abstandsbestimmungsvorrichtung verbunden. Der Halter 61 ist über das zweite Teil 6111 mit einem Gelenk eines Schwenkmechanismus 62 verbunden, der ein Schwenken des Halters 61 und damit einer Ausrichtung der Markierungskugeln 6 relativ zum dem Grundkörper 9 ermöglicht. Dies hat zum einen den Sinn, dass die Markierungskugeln 6 so positioniert werden können, dass sie gut von der Messkamera (vergleiche Figuren 1 und 2) erfasst werden können. Zum anderen können die Markierungskugeln auf reproduzierbare Weise in vordefinierte Positionen gebracht werden. Insbesondere können die Markierungskugeln so positioniert werden, dass sie das Arbeiten mit dem Endoskop möglichst wenig behindern. Die Position der Markierungskugeln kann beim Kalibrieren des Endoskops (siehe oben) ermittelt werden.

Der Schwenkmechanismus 62 weist ein zylindrisches Element 622 auf, an dem das Teil 6111 des Halters 61 festgelegt ist (an der Mantelfläche des Elementes 622). An einer Stirnseite (die dem Endoskopschaft 31 abgewandt ist) weist das zylindrische Element 622 ein Gewinde auf, in das ein Stift 6231 einer Arretierkappe 623 eingeschraubt ist (vgl. Fig. 4). Die Arretierkappe 623 durchgreift zudem eine Bohrung in einem zwischen der Arretierkappe und dem zylindrischen Element angeordneten Wandelement 95 des Grundkörpers 9, so dass das Teil 6111 und damit die Markierungskugeln 6 an dem Grundkörper festgelegt sind, jedoch entlang der Achse des Stiftes 6231 der Arretierkappe gedreht werden kann, wenn die Arretierkappe gelöst ist. Insbesondere verläuft der Stift 6231 in Richtung der optischen Achse (bzw. des Schaftes 31) des Endoskops 3, so dass die Markierungskugeln 6 (d. h. die Ebene, entlang der die Markierungskugeln angeordnet sind) um eine zur optischen Achse des Endoskops 3 parallel verlaufende Achse gedreht werden können.

### Bezugszeichenliste

- 1: Kalibriervorrichtung
- 2: Abstandsbestimmungsvorrichtung
- 3: Endoskop
- 4: Träger
- 5: Kalibriermuster
- 6: Markierungskugel
- 7: Messkamera
- 8: Lichtfleck
- 9: Grundkörper
- 21: Rohr
- 22: Lichtleitfaser
- 23: Adapterrohr
- 24: Crimphülse
- 25: Klebstoff
- 26: Schutzschlauch
- 27: Kapillare
- 28: Tülle
- 29: Stablinse
- 31: Endoskopschaft
- 32: Abbildungsoptik
- 41: Führung
- 51: Kalibrierpunkt
- 61: Halter
- 62: Schwenkmechanismus
- 91: zweiter Kanal
- 95: Wandabschnitt
- 200: Stecker
- 211: Stirnseite des Rohrendes
- 221: erster Abschnitt
- 222: zweiter Abschnitt
- 311: Spitze des Endoskopschaftes
- 411: Backe
- 622: Zylinderelement
- 623: Arretierkappe
- 921: Aussparung
- 922: Führungselement
- 991: Bohrung
- 2211: Ende der Lichtleitfaser
- 4111: Nut
- 6110: erstes Teil
- 6111: zweites Teil
- 6231: Stift
- 9221,9222: Ausnehmung

## Patentansprüche

1. Verfahren zum Kalibrieren einer Abstandsbestimmungsvorrichtung zum Bestimmen eines Abstandes oder Ortsvektors zwischen einem optischen System umfassend eine Optik und eine Kamera und einem Objekt, mit den Schritten:
a) Bereitstellen eines Erfassungssystems, das eine Mehrzahl von Markierungselementen (6) sowie eine Messkamera (7) zum Erfassen der räumlichen Position der Markierungselemente (6) umfasst;
b) Anordnen der Markierungselemente (6) an dem optischen System (3) und/oder der Abstandsbestimmungsvorrichtung (2) umfassend Mittel zum Projizieren einer Lichtstruktur (8) auf ein Kalibriermuster (5);
c) Bereitstellen eines Trägers (4), der das Kalibriermuster (5) ausformt;
d) Projizieren einer Lichtstruktur auf den Träger mittels der Abstandsbestimmungsvorrichtung;
e) Erfassen der Markierungselemente (6) und Erfassen des Kalibriermusters (5) und an dem Träger (4) angeordneter Trägermarkierungselemente mittels der Messkamera (7);
f) Erfassen der Lichtstruktur (8) und des Kalibriermusters (5) unter Verwendung der Optik und der Kamera des optischen Systems;
g) Ermitteln der räumlichen Position der Markierungselemente (6) und damit des optischen Systems (3) unter Verwendung von Daten der Messkamera (7);
h) Ermitteln von Koordinaten des Kalibriermusters (5) in einem ersten, der Messkamera (7 ) zugeordneten Koordinatensystem unter Verwendung von Daten der Messkamera (7);
i) Ermitteln von Koordinaten des Bildes der Lichtstruktur (8) und des Kalibriermusters (5) in einem zweiten, der Bildebene der Kamera des optischen Systems zugeordneten Koordinatensystem; und
j) Kalibrieren der Abstandsbestimmungsvorrichtung (2) unter Verwendung der ermittelten Koordinaten sowie der ermittelten räumlichen Position des optischen Systems (3), wobei die Koordinaten der Lichtstruktur (8) in dem ersten Koordinatensystem anhand der Koordinaten des Kalibriermusters (5) in dem ersten Koordinatensystem, der Koordinaten des Kalibriermusters (5) in dem zweiten Koordinatensystem und der Koordinaten der Lichtstruktur (8) in dem zweiten Koordinatensystem bestimmt werden.

2. Verfahren nach Anspruch 1, **dadurch gekennzeichnet, dass** sich das optische System (3) während der Schritte d) - i) in einer ersten Position relativ zu dem Träger (4) mit dem Kalibriermuster (5) befindet, das optische System (3) nach Durchführen der Schritte d) - i) aus der ersten in eine zweite Position relativ zu dem Träger (4) gebracht und die Schritte d) - i) abermals durchgeführt werden, wobei für die erste und die zweite Position des optischen Systems jeweils erste Koordinaten des Kalibriermusters (5) in dem ersten Koordinatensystem und zweite Koordinaten des Bildes des Kalibriermusters (5) und der Lichtstruktur (8) in dem zweiten Koordinatensystem bestimmt und die Abstandsbestimmungsvorrichtung anhand dieser Koordinaten unter Berücksichtigung der jeweils ermittelten räumlichen Position des optischen Systems (3) kalibriert wird.

3. Verfahren nach Anspruch 1 oder 2, **dadurch gekennzeichnet, dass** eine an die ersten und zweiten Koordinaten des Kalibriermusters (5) angepasste Näherungsabbildung bestimmt wird, mit der Koordinaten im zweiten Koordinatensystem in Koordinaten im zweiten Koordinatensystem abbildbar sind, wobei unter Anwendung der Näherungsabbildung auf die zweiten Koordinaten der Lichtstruktur (8) die ersten Koordinaten der Lichtstruktur (8) bestimmt werden.

4. Verfahren nach einem der vorhergehenden Ansprüche, **dadurch gekennzeichnet, dass** das Kalibriermuster eine Mehrzahl von zueinander beabstandeten Strukturen aufweist, wobei
- jeweils die ersten und die zweiten Koordinaten der Strukturen des Kalibriermusters (5) bestimmt werden,
- die zweiten Koordinaten der Lichtstruktur (8) bestimmt werden; und
- unter Verwendung der zweiten Koordinaten der Lichtstruktur (8) und durch Interpolation der ersten Koordinaten der Strukturen des Kalibriermusters (5) die ersten Koordinaten der Lichtstruktur (8) bestimmt werden.

5. Verfahren nach einem der vorhergehenden Ansprüche, **dadurch gekennzeichnet, dass** das Kalibrieren gemäß Schritt j) das Erstellen einer Kalibriertabelle umfasst, in der für die erste und die zweite Position des optischen Systems (3) jeweils die ermittelten ersten und zweiten Koordinaten der Lichtstruktur (8) gegenübergestellt sind.

6. Verfahren nach einem der vorhergehenden Ansprüche, **dadurch gekennzeichnet, dass** für die unterschiedlichen Positionen des optischen Systems (3) die ersten Koordinaten der Lichtstruktur (8) durch eine erste Näherungsfunktion und/oder die zweiten Koordinaten der Lichtstruktur (8) durch eine zweite Näherungsfunktion dargestellt werden.

7. Verfahren nach Anspruch 6, **dadurch gekennzeichnet, dass** eine Übertragungsfunktion zum Abbilden der ersten Näherungsfunktion auf die zweiten Näherungsfunktion bestimmt wird, wobei mit Hilfe dieser Übertragungsfunktion aus den zweiten Koordinaten der Lichtstruktur (8) die ersten Koordinaten der Lichtstruktur (8) ermittelbar sind.

8. Verfahren nach Anspruch 6 oder 7, **dadurch gekennzeichnet, dass** die erste und/oder die zweite Näherungsfunktion in Form einer Geraden oder eines Polynoms bestimmt wird.

9. Verfahren nach einem der Ansprüche 6 bis 8, **dadurch gekennzeichnet, dass** die erste Näherungsfunktion bestimmt und das optische System (3) in mindestens eine weitere Position gebracht wird, wobei das Kalibrieren gemäß Schritt j) für die mindestens eine weitere Position die folgenden Schritte umfasst:
i. Bestimmen der zweiten Koordinaten der Lichtstruktur (8),
ii. Bestimmen einer Geraden im ersten Koordinatensystem, die durch die Position der Lichtstruktur (8) in der Bildebene des optischen Systems (3) und durch ein optisches Zentrum des optischen Systems (3) verläuft, und
iii. Bestimmen der ersten Koordinaten der Lichtstruktur (8) durch Ermitteln der Koordinaten des Schnittpunktes der Geraden mit der ersten Näherungsfunktion.

10. Verfahren nach Anspruch 9, **dadurch gekennzeichnet, dass** die erste Näherungsfunktion als Gerade darstellbar ist und die ersten Koordinaten der Lichtstruktur (8) in dem ersten Koordinatensystem durch Ermitteln der Koordinaten des Schnittpunktes der gemäß Schritt ii) bestimmten Geraden mit der die erste Näherungsfunktion darstellenden Geraden oder durch Ermitteln der Koordinaten eines Punktes eines Gemeinlots in Bezug auf die beiden Geraden bestimmt werden.

11. Verfahren nach Anspruch 9 oder 10, **dadurch gekennzeichnet, dass** das Kalibrieren gemäß Schritt j) das Erstellen einer Kalibriertabelle umfasst, in der sowohl die für die erste und die zweite Position ermittelten ersten Koordinaten der Lichtstruktur (8) als auch die für die mindestens eine weitere Position des optischen Systems (3) nach den Ansprüchen 9 und 10 ermittelten ersten Koordinaten der Lichtstruktur (8) den jeweiligen zweiten Koordinaten der Lichtstruktur (8) gegenübergestellt sind.

12. Verfahren nach einem der Ansprüche 2 bis 11, **dadurch gekennzeichnet, dass** anhand der bestimmten Koordinaten des Kalibriermusters (5) und der Lichtstruktur (8) sowie der bestimmten räumlichen Position des optischen Systems (3) jeweils für die erste und die zweite Position des optischen Systems (3) ein Ortsvektor zwischen einem Punkt der auf den Träger (4) projizierten Lichtstruktur (8) und einem Punkt des optischen Systems (3) bestimmt wird und das Kalibrieren gemäß Schritt j) das Zuordnen dieses Ortsvektors zu den ermittelten zweiten Koordinaten der Lichtstruktur (8) umfasst.

13. Verfahren nach einem der vorhergehenden Ansprüche, **dadurch gekennzeichnet, dass** das Ermitteln der räumlichen Position des optischen Systems (3) mit Hilfe der Markierungselemente (6) das Bestimmen einer Position einer Vorderseite des optischen Systems (3) umfasst, die bei Verwendung des optischen Systems (3) zum Abbilden eines Objektes diesem Objekt zuzuwenden ist.

14. Kalibriervorrichtung zum Kalibrieren einer Abstandsbestimmungsvorrichtung zum Bestimmen eines Abstandes oder Ortvektors zwischen einem optischen System umfassend eine Optik und eine Kamera und einem Objekt, zum Durchführen eines Verfahrens gemäß einem der vorhergehenden Ansprüche, mit
a) einem Träger (4), der ein Kalibriermuster (5) ausformt;
b) Mitteln zur Projektion einer Lichtstruktur (8) auf das Kalibiermuster (5);
c) einem Erfassungssystem, das eine Mehrzahl von an dem optischen System (3) und/oder der Abstandsbestimmungsvorrichtung (2) angeordneten Markierungselementen (6) sowie eine Messkamera (7) zum Erfassen der räumlichen Position der Markierungselemente (6) und an dem Träger (4) angeordneter Trägermarkierungselemente umfasst;
d) Positionsermittlungsmitteln zum Ermitteln der räumlichen Position des optischen Systems (3) anhand der Daten der Messkamera (7) sowie Positionsermittlungsmitteln umfassend die Kamera des optischen Systems zur Erfassung der Lichtstruktur (8) und des Kalibriermusters (5);
e) Koordinatenermittlungsmitteln zum Ermitteln von Koordinaten des Kalibriermusters (5) in einem ersten, der Messkamera (7) zugeordneten Koordinatensystem, von Koordinaten eines Bildes des Kalibriermusters und einer unter Verwendung von der Abstandsbestimmungsvorrichtung (2) zugehörigen Mitteln zum Projizieren projizierten Lichtstruktur (8) in einem zweiten, der Bildebene einer Kamera des optischen Systems (3) zugeordneten Koordinatensystem; sowie
f) Kalibrierungsmitteln zum Kalibrieren der Abstandsbestimmungsvorrichtung (2) anhand der ermittelten Koordinaten sowie der ermittelten räumlichen Position des optischen Systems (3), wobei
g) die Kalibrierungsmittel ausgebildet sind, die Koordinaten der Lichtstruktur (8) in dem ersten Koordinatensystem anhand der Koordinaten des Kalibriermusters (5) in dem ersten Koordinatensystem, der Koordinaten des Kalibriermusters (5) in dem zweiten Koordinatensystem und der Koordinaten der Lichtstruktur (8) in dem zweiten Koordinatensystem zu bestimmen.

15. Kalibriervorrichtung nach Anspruch 14, **dadurch gekennzeichnet, dass** das Kalibriermuster (5) auf den Träger (4) aufgeklebt, aufgedruckt und/oder in den Träger (4) eingeprägt ist.

## Claims

1. A method for calibrating a distance determining device for determining a distance or position vector between an optical system comprising an optics and a camera and an object, comprising the steps:
a) providing a detecting system, which comprises a plurality of marking elements (6) and a measuring camera (7) for detecting the spatial position of the marking elements (6);
b) arranging the marking elements (6) on the optical system (3) and/or the distance determining device (2) comprising means for projecting a light structure (8) onto a calibration pattern (5);
c) providing a carrier (4) which forms the calibration pattern (5);
d) projecting a light structure onto the carrier by means of the distance determining device;
e) detecting the marking elements (6) and detecting the calibration pattern (5) and carrier marking elements arranged on the carrier (4) by means of the measuring camera (7);
f) detecting the light structure (8) and the calibration pattern (5) using the optics and the camera of the optical system;
g) determining the spatial position of the marking elements (6) and thus of the optical system (3) using data of the measuring camera (7);
h) determining coordinates of the calibration pattern (5) in a first coordinate system associated with the measuring camera (7) using data of the measuring camera (7);
i) determining coordinates of an image of the light structure (8) and a calibration pattern (5) in a second coordinate system associated with the image plane of the camera of the optical system; and
j) calibrating the distance determining device (2) using the determined coordinates and the determined spatial position of the optical system (3), wherein the coordinates of the light structure (8) in the first coordinate system are determined using the coordinates of the calibration pattern (5) in the first coordinate system, the coordinates of the calibration pattern (5) in the second coordinate system, and the coordinates of the light structure (8) in the second coordinate system.

2. The method according to claim 1, **characterized in that** the optical system (3) is in a first position relative to the carrier (4) with the calibration pattern (5) during the steps d) - i), the optical system (3) is brought from the first into a second position relative to the carrier (4) after carrying out the steps d) - i) and the steps d) - i) are carried out again, wherein first coordinates of the calibration pattern (5) in the first coordinate system and second coordinates of the image of the calibration pattern (5) and of the light structure (8) in the second coordinate system are determined for the first and the second position of the optical system, respectively, and the distance determination device is calibrated on the basis of these coordinates, taking into account the respective determined spatial position of the optical system (3).

3. The method according to claim 1 or 2, **characterized in that** a proximity image adapted to the first and second coordinates of the calibration pattern (5) is determined with which coordinates of the second coordinate system can be imaged in coordinates in the second coordinate system, wherein the first coordinates of the light structure (8) are determined by applying the proximity image on the second coordinates of the light structure (8).

4. The method according to any of the preceding claims, **characterized in that** the calibration pattern comprises a plurality of structures distanced from each other, wherein
- the first and the second coordinates of the structure of the calibration pattern (5) are determined, respectively,
- the second coordinates of the light structure (8) are determined; and
- the first coordinates of the light structure (8) are determined using the second coordinates of the light structure (8) and by interpolation of the first coordinates of the structures of the calibration pattern (5).

5. The method according to any of the preceding claims, **characterized in that** the calibration according to step j) comprises the generation of a calibration table in which, for the first and the second position of the optical system (3), the determined first and second coordinates of the light structure (8) are contrasted, respectively.

6. The method according to any of the preceding claims, **characterized in that** the first coordinates of the light structure (8) are illustrated by a first proximity function and/or the second coordinates of the light structure (8) are illustrated by a second proximity function for the different positions of the optical system (3).

7. The method according to claim 1, **characterized in that** a transfer function for imaging the first proximity function onto the second proximity function is determined, wherein the first coordinates of the light structure (8) can be determined from the second coordinates of the light structure (8) by means of this transfer function.

8. The method according to claim 1 or 2, **characterized in that** the first and/or the second proximity function is determined in form of a line or a polynomial.

9. The method according to any of claims 6 to 8, **characterized in that** the first proximity function is determined and the optical system is brought into at least one further position, wherein the calibration according to step j) for the at least one further position comprises the following steps:
i. determining the second coordinates of the light structure (8),
ii. determining a line in the first coordinate system which continues through the position of the light structure (8) in the image plane of the optical system (3) and through an optical center of the optical system (3), and
iii. determining the first coordinates of the light structure (8) by determining the coordinates of the intersection point of the line with the first proximity function.

10. The method according to claim 9, **characterized in that** the first proximity function can be represented as a line and the first coordinates of a light structure (8) can be determined in the first coordinate system by determining the coordinates of the intersection point of the line determined according to step ii) with the line representing the first proximity function or by determining the coordinates of a point of a common perpendicular relative to both lines.

11. The method according to claim 9 or 10, **characterized in that** the calibration according to step j) comprises generating a calibration table in which the first coordinates of the light structure (8) determined for the first and the second position as well as also the first coordinates of the light structure (8) determined for the at least one further position of the optical system (3) according to claims 9 and 10 are contrasted to the respective second coordinates of the light structure (8).

12. The method according to any of claims 2 to 11, **characterized in that** by means of the determined coordinates of the calibration pattern (5) and the light structure (8) and the determined spatial position of the optical system (3) a position vector between a point of the light structure (8) projected onto the carrier (3) and a point of the optical system (3) is determined for the first and the second position of the optical system (3), respectively, and the calibration according to step j) comprises the assigning of this position vector to the determined second coordinates of the light structure (8).

13. The method according to any of the preceding claims, **characterized in that** the determining of the spatial position of the optical system (3) by means of the marking elements (6) comprises determining a position of a front side of the optical system (3), which when using the optical system (3) for imaging an object is to be turned towards this object.

14. A calibrating device for calibrating a distance determining device for determining a distance or a position vector between an optical system comprising an optics and a camera and an object, for carrying out a method according to any of the preceding claims, comprising
a) a carrier (4) which forms a calibration pattern (5);
b) means for projecting a light structure (8) onto the calibration pattern (5);
c) a detection system which comprises a plurality of marking elements (6) arranged on the optical system (3) and/or the distance determining device (2) and a measuring camera (7) for detecting the spatial position of the marking elements (6) and carrier marking elements arranged on the carrier (4);
d) position determining means for determining the spatial position of the optical system (3) by means of the data of the measuring camera (7) as well as position determining means comprising the camera of the optical system for the detection of the light structure (8) and the calibration pattern (5);
e) coordinates determining means for determining coordinates of the calibration pattern (5) in a first coordinate system associated to the measuring camera (7), of coordinates of an image of the calibration pattern and a light structure (8) projected using means for projection associated with distance determining device (2) in a second coordinate system associated to the image plane of a camera of the optical system (3); as well as
f) calibration means for calibrating the distance determining device (2) by means of the determined coordinates and the determined spatial position of the optical system (3), wherein
g) the calibration means are configured to determine the coordinates of the light structure (8) in the first coordinate system using the coordinates of the calibration pattern (5) in the first coordinate system, the coordinates of the calibration pattern (5) in the second coordinate system, and the coordinates of the light structure (8) in the second coordinate system.

15. The calibration device according to claim 14, **characterized in that** the calibration pattern (5) is glued to the carrier (4), printed on and/or engraved into the carrier (4).

## Revendications

1. Dispositif d'étalonnage d'un dispositif de mesure de distance pour mesurer une distance ou un vecteur de position entre un système optique comprenant une optique et une caméra et un objet, comprenant les étapes suivantes :
a) la mise à disposition d'un système de détection comprenant une pluralité d'éléments de marquage (6) ainsi qu'une caméra de mesure (7) pour détecter la position spatiale des éléments de marquage (6);
b) la mise en place des éléments de marquage (6) sur le système optique (3) et/ou sur le dispositif de mesure de distance (2) comprenant des moyens pour projeter une structure lumineuse (8) sur un motif d'étalonnage (5);
c) la mise à disposition d'un support (4) formant le motif d'étalonnage (5);
d) la projection d'une structure lumineuse sur le support à l'aide du dispositif de mesure de distance;
e) la détection des éléments de marquage (6) et la détection du motif d'étalonnage (5) et des éléments de marquage disposés sur le support (4) à l'aide de la caméra de mesure (7);
f) la détection de la structure lumineuse (8) et du motif d'étalonnage (5) en utilisant l'optique et la caméra du système optique;
g) la détermination de la position spatiale des éléments de marquage (6) et donc du système optique (3) en utilisant des données de la caméra de mesure (7);
h) la détermination des coordonnées du motif d'étalonnage (5) dans un premier système de coordonnées attribué à la caméra de mesure (7) en utilisant des données de la caméra de mesure (7);
i) la détermination de coordonnées de l'image de la structure lumineuse (8) et du motif d'étalonnage (5) dans un deuxième système de coordonnées attribué au plan image de la caméra du système optique; et
j) l'étalonnage du dispositif de mesure de distance (2) en utilisant les coordonnées déterminées ainsi que la position spatiale identifiée du système optique (3), les coordonnées de la structure lumineuse (8) dans le premier système de coordonnées étant déterminées à l'aide des coordonnées du motif d'étalonnage (5) dans le premier système de coordonnées, à l'aide des coordonnées du motif d'étalonnage (5) dans le deuxième système de coordonnées et à l'aide des coordonnées de la structure lumineuse (8) dans le deuxième système de coordonnées.

2. Procédé selon la revendication 1, **caractérisé en ce que** le système optique (3) se trouve pendant les étapes d) à i) dans une première position relative au support (4) avec le motif d'étalonnage (5), ce système optique (3) est, une fois les étapes d) à i) réalisées, amené de la première position à une deuxième position par rapport au support (4) et les étapes d) à i) sont réalisées à nouveau, des premières coordonnées du motif d'étalonnage (5) étant déterminées dans le premier système de coordonnées, et des deuxièmes coordonnées de l'image du motif d'étalonnage (5) et de la structure lumineuse (8) dans le second système de coordonnées, pour la première et la deuxième position du système optique, et le dispositif de mesure de distance étant étalonné à l'aide de ces coordonnées respectives en tenant compte des différentes positions spatiales déterminées pour le système optique (3).

3. Procédé selon la revendication 1 ou 2, **caractérisé en ce que** l'on définit une représentation d'approximation ajustée aux premières et deuxièmes coordonnées du motif d'étalonnage (5) à l'aide de laquelle des coordonnées dans le deuxième système de coordonnées peuvent être représentées dans des coordonnées dans le deuxième système de coordonnées, les premières coordonnées de la structure lumineuse (8) étant déterminées en appliquant la représentation d'approximation aux deuxièmes coordonnées de la structure lumineuse (8).

4. Procédé selon l'une des revendications précédentes, **caractérisé en ce que** le motif d'étalonnage présente une pluralité de structures espacées les unes des autres,
- les premières et les deuxièmes coordonnées des structures du motif d'étalonnage (5) étant respectivement déterminées,
- les deuxièmes coordonnées de la structure lumineuse (8) étant déterminées; et
- les premières coordonnées de la structure lumineuse (8) étant déterminées en utilisant les deuxièmes coordonnées de la structure lumineuse (8) et par interpolation des premières coordonnées des structures du motif d'étalonnage (5).

5. Procédé selon l'une des revendications précédentes, **caractérisé en ce que** l'étalonnage selon l'étape j) comprend la création d'un tableau d'étalonnage dans lequel les premières et les deuxièmes coordonnées de la structure lumineuse (8) respectivement déterminées sont comparées pour la première et la deuxième position du système optique (3).

6. Procédé selon l'une des revendications précédentes, **caractérisé en ce que** les premières coordonnées de la structure lumineuse (8) sont représentées à travers une première fonction d'approximation, et/ou les deuxièmes coordonnées de la structure lumineuse (8) à travers une deuxième fonction d'approximation, pour les différentes positions du système optique (3).

7. Procédé selon la revendication 6, **caractérisé en ce que** l'on définit une fonction de transfert pour représenter la première fonction d'approximation sur la deuxième fonction d'approximation, cette fonction de transfert permettant de déterminer les premières coordonnées de la structure lumineuse (8) à partir des deuxièmes coordonnées de la structure lumineuse (8).

8. Procédé selon la revendication 6 ou 7, **caractérisé en ce que** la première et/ou la deuxième fonction d'approximation sont déterminées sous la forme d'une droite ou d'un polynôme.

9. Procédé selon l'une des revendications 6 à 8, **caractérisé en ce que** la première fonction d'approximation est déterminée et le système optique (3) amené dans au moins une position supplémentaire, l'étalonnage selon l'étape j) pour ladite au moins une position supplémentaire consistant en les étapes suivantes:
i. la détermination des deuxièmes coordonnées de la structure lumineuse (8),
ii. la détermination d'une droite dans le premier système de coordonnées qui s'étend à travers la position de la structure lumineuse (8) dans le plan image du système optique (3) et à travers un centre optique du système optique (3), et
iii. la détermination des premières coordonnées de la structure lumineuse (8) par détermination des coordonnées du point d'intersection de la droite avec la première fonction d'approximation.

10. Procédé selon la revendication 9, **caractérisé en ce que** la première fonction d'approximation peut être représentée sous la forme d'une droite et les premières coordonnées de la structure lumineuse (8) dans le premier système de coordonnées sont déterminées par détermination des coordonnées du point d'intersection de la droite déterminée selon l'étape ii) avec la droite représentant la première fonction d'approximation ou par détermination des coordonnées d'un point d'une perpendiculaire commune par rapport aux deux droites.

11. Procédé selon la revendication 9 ou 10, **caractérisé en ce que** l'étalonnage selon l'étape j) comprend la création d'un tableau d'étalonnage dans lequel aussi bien les premières coordonnées de la structure lumineuse (8) déterminées pour la première et la deuxième position que les premières coordonnées de la structure lumineuse (8) déterminées selon les revendications 9 et 10 pour ladite au moins une position supplémentaire du système optique (3) sont comparées aux deuxièmes coordonnées respectives de la structure lumineuse (8).

12. Procédé selon l'une des revendications 2 à 11, **caractérisé en ce qu'**à partir des coordonnées déterminées pour le motif d'étalonnage (5) et la structure lumineuse (8) ainsi que de la position spatiale déterminée pour le système optique (3), on détermine respectivement pour la première et la deuxième position du système optique (3) un vecteur de position entre un point de la structure lumineuse (8) projetée sur le support (4) et un point du système optique (8), et l'étalonnage selon l'étape j) comprend l'attribution de ce vecteur de position aux deuxièmes coordonnées déterminées pour la structure lumineuse (8).

13. Procédé selon l'une des revendications précédentes, **caractérisé en ce que** la détermination de la position spatiale du système optique (3) à l'aide des éléments de marquage comprend la détermination d'une position d'une face avant du système optique (3) qui, lors de l'utilisation du système optique (3) pour représenter un objet, doit être tournée vers cet objet.

14. Dispositif d'étalonnage d'un dispositif de mesure de distance pour mesurer une distance ou un vecteur de position entre un système optique comprenant une optique et une caméra et un objet pour réaliser un procédé selon l'une des revendications précédentes, comprenant
a) un support (4) formant un motif d'étalonnage (5);
b) des moyens pour projeter une structure lumineuse (8) sur le motif d'étalonnage (5);
c) un système de détection comportant une pluralité d'éléments de marquage (6) disposés sur le système optique (3) et/ou le dispositif de mesure de distance (2), ainsi qu'une caméra de mesure pour détecter la position spatiale des éléments de marquage (6) et d'éléments de marquage (6) disposés sur le support (4);
d) moyens de détermination de position pour déterminer la position spatiale du système optique (3) à partir des données de la caméra de mesure (7) ainsi que des moyens de détermination de position comprenant la caméra du système optique pour la détection de la structure lumineuse (8) et du motif d'étalonnage (5);
e) des moyens de détermination de coordonnées pour déterminer des coordonnées du motif d'étalonnage (5) dans un premier système des coordonnées associé à la caméra de mesure (7), des coordonnées d'une image du motif d'étalonnage ainsi que, en utilisant des moyens associés au dispositif de mesure de distance (2) et destinés à la projection, une structure lumineuse (3) projetée dans un deuxième système de coordonnées attribué au plan image d'une caméra d'un système optique (3), ainsi que
f) des moyens d'étalonnage pour étalonner le dispositif de mesure de distance (2) à partir des coordonnées déterminées et de la position spatiale déterminée pour le système optique (3),
g) les moyens d'étalonnage étant conçus pour déterminer les coordonnées de la structure lumineuse (8) dans le premier système de coordonnées à partir des coordonnées du motif d'étalonnage (5) dans le premier système de coordonnées, des coordonnées du motif d'étalonnage (5) dans le deuxième système de coordonnées et des coordonnées de la structure lumineuse (8) dans le deuxième système de coordonnées.

15. Procédé selon la revendication 14, **caractérisé en ce que** le motif d'étalonnage (5) est collé, imprimé et/ou gravé sur le support (4).
